# EUROPEAN PATENT SPECIFICATION

(11) **EP 1 237 619 B1**
(45) Date of publication and mention of the grant of the patent: **15.09.2004**
(21) Application number: 00982292.5
(22) Date of filing: 30.11.2000
(51) Int. Cl.: A61N 1/05, A61N 1/34

(54) **PERCUTANEOUS ELECTRICAL THERAPY SYSTEM AND ELECTRODE**
SYSTEM UND ELEKTRODE ZUR PERKUTANEN ELEKTRISCHEN THERAPIE
ELECTRODE ET SYSTEME DE THERAPIE ELECTRIQUE PERCUTANEE

(30) Priority: 01.12.1999 US 452477; 01.12.1999 US 452663; 01.12.1999 US 452508; 01.12.1999 US 451795; 01.12.1999 US 451799; 01.12.1999 US 452510; 01.12.1999 US 451800; 01.12.1999 US 451796; 01.12.1999 US 451547
(43) Date of publication of application: 11.09.2002
(73) Proprietor: MEAGAN MEDICAL, INC., Reno, Nevada 89509 (US)
(72) Inventor: BISHAY, Jon, M., Woodinville, WA 98072 (US); LEONARD, Paul, C., Woodinville, WA 98072 (US); LEYDE, Kent, W., Redmond, WA 98053 (US)
(74) Representative: Grünecker, Kinkeldey, Stockmair & Schwanhäusser Anwaltssozietät
(86) International application number: PCT/US2000/032559
(87) International publication number: WO 2001/039829

(56) References cited:
- FR-A- 2 500 745
- US-A- 4 262 672
- US-A- 4 408 617
- US-A- 5 702 359
- US-A- 5 873 849

## Description

### TECHNICAL FIELD

This invention relates generally to percutaneous electrical therapy systems for medical use.

### BACKGROUND OF THE INVENTION

Electrical therapy has long been used in medicine to treat pain and other conditions. For example, transcutaneous electrical nerve stimulation (TENS) systems deliver electrical energy through electrode patches placed on the surface of a patient's skin to treat pain in tissue beneath and around the location of the patches. The efficacy of TENS systems in alleviating pain is questionable at best, however.

More recently, a technique in which electrodes are placed through the patient's skin into the target tissue has been proposed. Percutaneous Neuromodulation Therapy ("PNT") (also sometimes called Percutaneous Electrical Nerve Stimulation or "PENS") using percutaneously placed electrodes achieves significantly better pain relief results than TENS treatments using skin surface electrodes. This therapy is described in Ghoname *et al*., "Percutaneous Electrical Nerve Stimulation for Low Back Pain," JAMA 281:818-23 (1999); Ghoname *et al*., "The Effect of Stimulus Frequency on the Analgesic Response to Percutaneous Electrical Nerve Stimulation in Patients with Chronic Low Back Pain," Anesth. Analg. 88:841-6 (1999); Ahmed *et al*., "Percutaneous Electrical Nerve Stimulation (PENS): A Complementary Therapy for the Management of Pain Secondary to Bony Metastasis," Clinical Journal of Pain 14:320-3 (1998); and Ahmed *et al*., "Percutaneous Electrical Nerve Stimulation: An Alternative to Antiviral Drugs for Herpes Zoster," Anesth. Analg. 87:911 -4 (1998). The contents of these references are incorporated herein by reference. US-A-5 702 359 discloses an electrode apparatus comprising needle electrodes for the application of electroporation to a portion of the body of a patient.

Thus far, PNT practitioners have used percutaneously placed acupuncture needles attached to waveform generators via cables and alligator clips to deliver the therapy to the patient. This arrangement and design of electrodes and generator is far from optimal. For example, the prior art has not addressed the issue of how to control the entry angle of percutaneous electrodes used in PNT and other electrical therapies, or how to prevent percutaneous electrodes from buckling when they are inserted into the skin. Another drawback with conventional arrangements is that they may not control the depth to which the percutaneous electrodes are inserted, and may not prevent the electrodes from being inadvertently withdrawn from the skin. Conversely, conventional electrodes may also be difficult or awkward to deliberately withdraw from the patients. Still another drawback is that the electrical connection to the electrode may be unreliable and difficult to use. The patient may also experience discomfort when the electrode is inserted into the skin. Still further, some conventional systems may permit the patient's caregiver and/or a bystander to inadvertently contact the sharp end of the electrode, for example, when inserting or withdrawing the electrode.

### SUMMARY OF THE INVENTION

The present invention is as defined in the appended set of claims. It is directed to apparatuses for administering percutaneous electrical therapy. In one aspect of the invention, the apparatus can include an electrode electrically connectable to a control unit to deliver electrical therapy to a patient during operation. The electrode can have a first end and a second end opposite the first end with the first end having a sharp point configured to be inserted into tissue of the patient. The apparatus can further include an electrode housing operatively coupled to the electrode and positioned to support the electrode during insertion of the electrode into the tissue. The housing can be positioned relative to the electrode to control the motion of and/or the access to the electrode.

The housing can include a channel disposed annularly about the electrode to engage and guide at least a portion of the electrode during operation as the electrode moves relative to the housing into the tissue. The housing can include a pressure element positioned adjacent to the electrode to provide pressure against the tissue adjacent to an electrode insertion point through which the electrode enters the tissue. The apparatus can further include an electrode actuator attached to the electrode and movable with the electrode relative to the housing, and an actuator tool removably attached to the actuator to move the actuator and the electrode relative to the tissue. The housing can include a limit stop positioned to engage the electrode actuator for stopping the motion of the electrode actuator when the electrode reaches a selected depth in the tissue.

In another aspect of the invention, the housing forms an interface with the skin of the patient when the housing is engaged with the skin so that the housing and the skin completely surround the sharp point of the electrode as the electrode moves into the tissue. The apparatus can further include a housing alignment member disposed on the tissue and adapted to mechanically interact with the housing to align the housing relative to the tissue. In a further aspect of this embodiment, the alignment member can include a patch adhesively attached to the patient's skin.

The invention is also directed to a percutaneous electrode remover that includes a housing configured to be held in a human hand. The housing can have an aperture at a distal end and an actuator configured to be engaged by the human hand. In one aspect of the invention, the actuator is movable relative to the housing between a first position with the actuator coupled to an electrode while the electrode is inserted in a patient, and a second position with the actuator coupled to the electrode and the electrode withdrawn through the aperture and completely into the housing.

The apparatus of the invention is used in a method for administering percutaneous electrical therapy to a patient. The method can include aligning an electrode housing with tissue of the patient, moving at least one of the electrode and the housing relative to the other to insert a sharp point of the electrode into the tissue, and controlling a motion of and/or access to the electrode with the housing. The method can further include applying an electrical current to the electrode while the electrode is inserted in the tissue.

In one aspect, the method can include guiding the electrode in an axial direction by engaging at least a portion of the electrode with walls of a channel disposed annularly about the electrode as the electrode moves relative to the housing. The method can also include halting movement of the electrode when the sharp point of the electrode reaches a selected depth in the tissue. The method can further include applying pressure to a skin of the patient adjacent to an electrode insertion point as the electrode is passed into the patient at the electrode insertion point.

Furthermore, the method can include grasping a housing of a percutaneous electrode remover, engaging the housing with tissue proximate to the percutaneous electrode while the percutaneous electrode is inserted in the tissue, and manipulating an actuator of the percutaneous electrode remover to couple the actuator to the percutaneous electrode. The method can further include activating the actuator to withdraw the percutaneous electrode from the tissue and into the housing.

### BRIEF DESCRIPTION OF THE DRAWINGS

Figures 1A-G are schematic renderings of a percutaneous electrical therapy system according to one embodiment of this invention.
Figure 1A shows electrode and angle of insertion assemblies wherein the electrode is in an undeployed and uninserted state.
Figure 1B shows the electrode and angle of insertion assemblies of Figure 1A during deployment but prior to insertion of the electrode into a patient's tissue.
Figure 1C shows the electrode and angle of insertion assemblies of Figure 1A during deployment and insertion of the electrode into the patient's tissue.
Figure 1D shows the electrode of Figure 1A inserted into the patient's tissue.
Figure 1E shows the electrode of Figure 1A attached to a control unit to provide percutaneous electrical therapy.
Figure 1F shows the electrode and angle of insertion assemblies of Figure 1A during undeployment but prior to removing the electrode from the patient's tissue.
Figure 1G shows the electrode and sharp point protection assemblies of Figure 1A during undeployment and after removing the electrode from the patient's tissue.
Figures 2A-E are schematic renderings of a percutaneous electrical therapy system according to another embodiment of this invention.

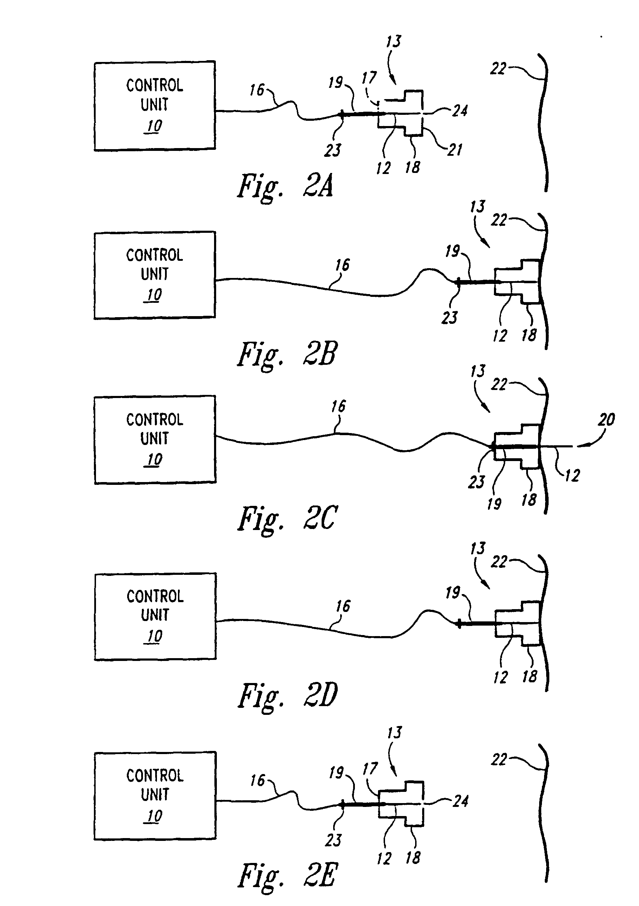

Figure 2A shows a percutaneous electrical therapy system with electrode and angle of insertion assemblies wherein the electrode is in an undeployed and uninserted state.
Figure 2B shows the percutaneous electrical therapy system of Figure 2A during deployment, but prior to insertion, of the electrode.
Figure 2C shows the percutaneous electrical therapy system of Figure 2A with the electrode in a deployed and inserted state.
Figure 2D shows the percutaneous electrical therapy system of Figure 2A during undeployment of the electrode.
Figure 2E shows the percutaneous electrical therapy system of Figure 2A after the electrode has been undeployed.

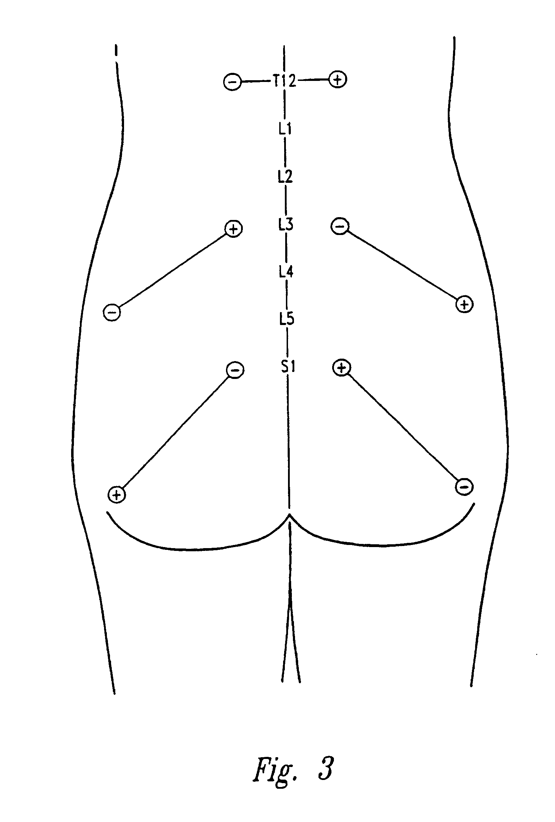

Figure 3 shows an electrode montage for use in percutaneous neuromodulation therapy to treat low back pain.

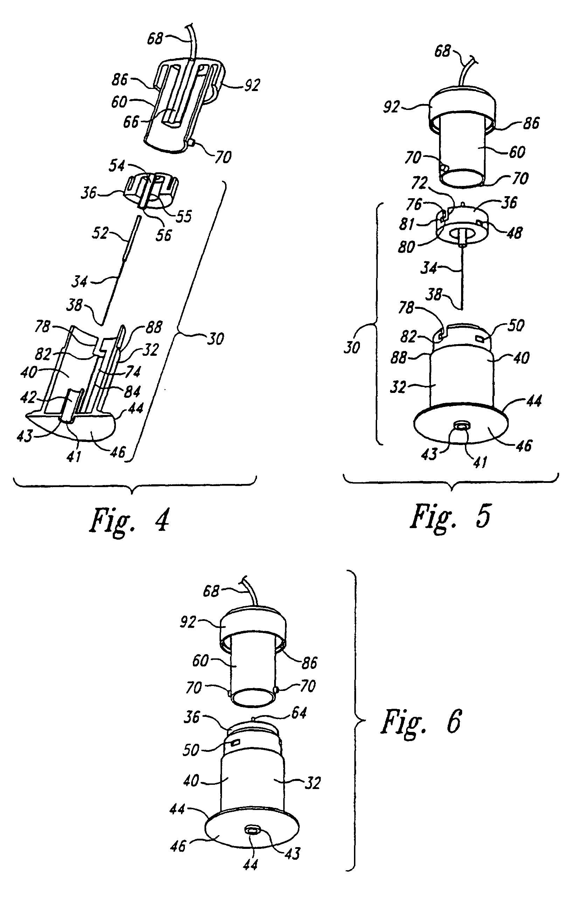

Figure 4 is an exploded sectional view of an electrode and angle of insertion assembly according to yet another embodiment of this invention.
Figure 5 is a partially exploded elevational view of the embodiment of Figure 4.
Figure 6 is an elevational view of the embodiment of Figure 4 showing the electrode and angle of insertion assemblies and an actuator tool.

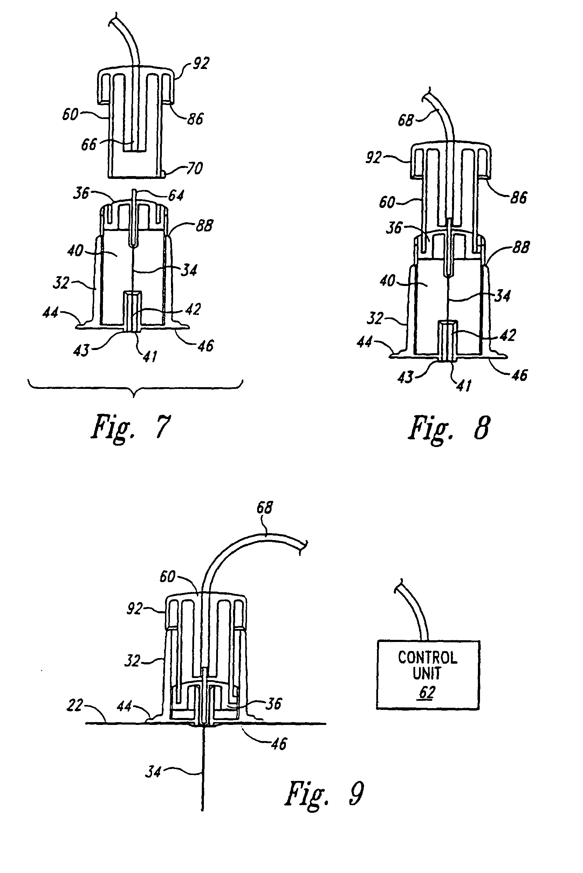

Figure 7 is a sectional view of the embodiment of Figure 4 showing the electrode and angle of insertion assemblies and an actuator tool.
Figure 8 is a sectional view of the embodiment of Figure 4 showing the actuator tool in engagement with the electrode and angle of insertion assemblies prior to insertion of the electrode into a patient's tissue.
Figure 9 is a sectional view of the embodiment of Figure 4 with the electrode in its deployed and inserted state.

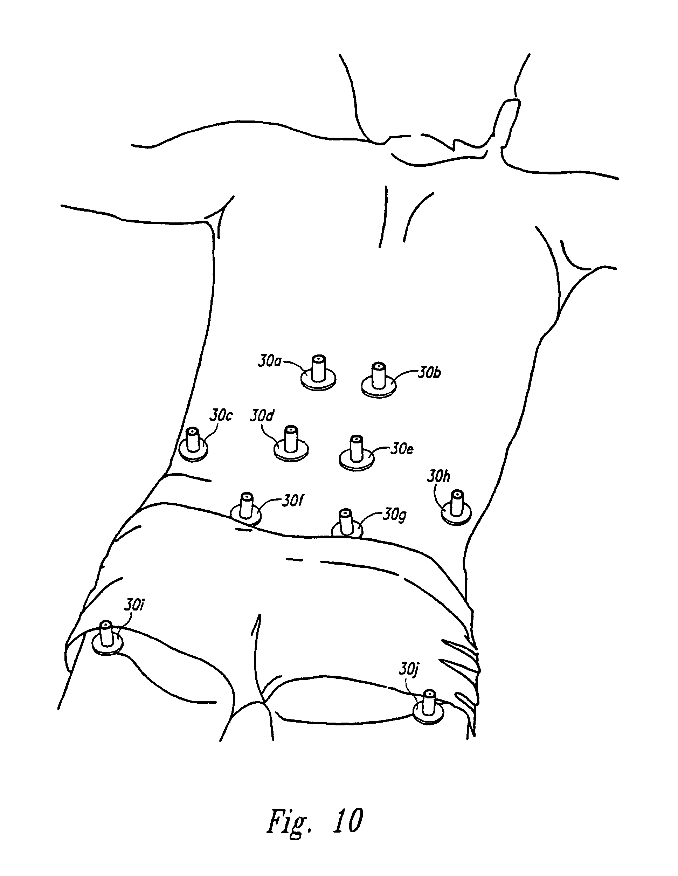

Figure 10 shows a montage for using the embodiment of Figure 4 to treat low back pain with the electrodes in a partially deployed but uninserted state.

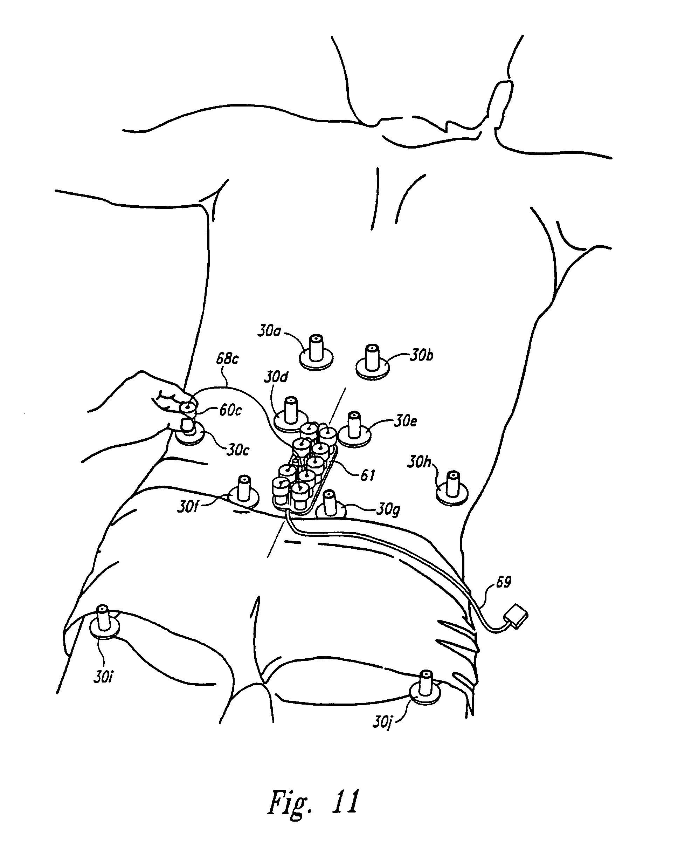

Figure 11 shows the electrode montage of Figure 10 at the beginning of the electrode insertion step.

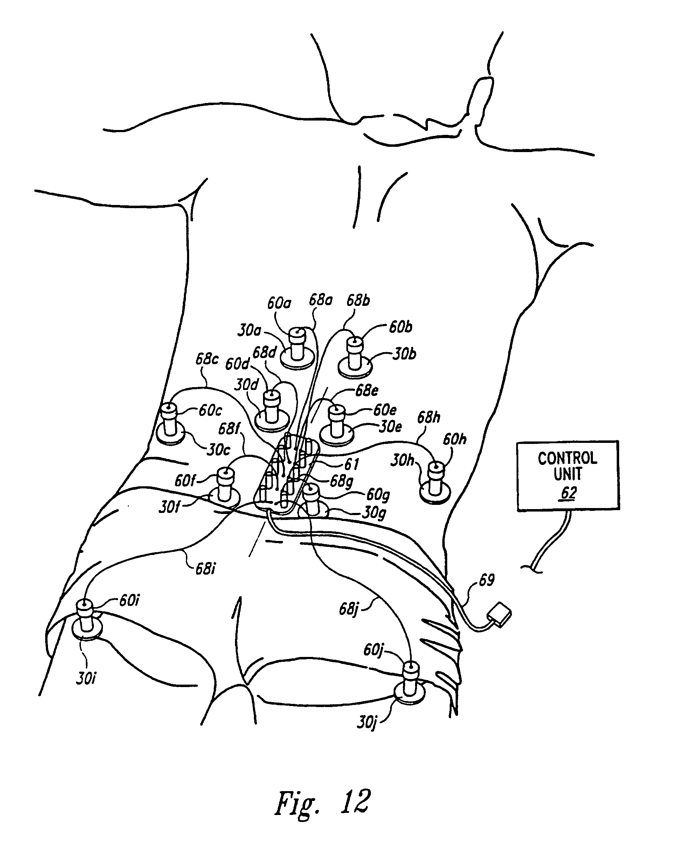

Figure 12 shows the electrode montage of Figure 10 with the electrodes deployed, inserted and attached to a control unit to provide electrical therapy to the patient.

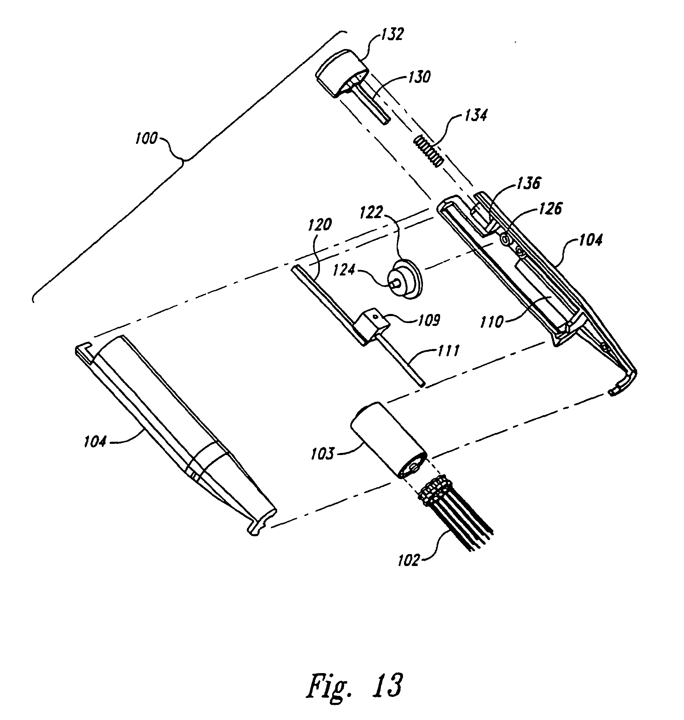

Figure 13 is an exploded view of an electrode introducer and angle of insertion assembly of yet another embodiment of this invention.

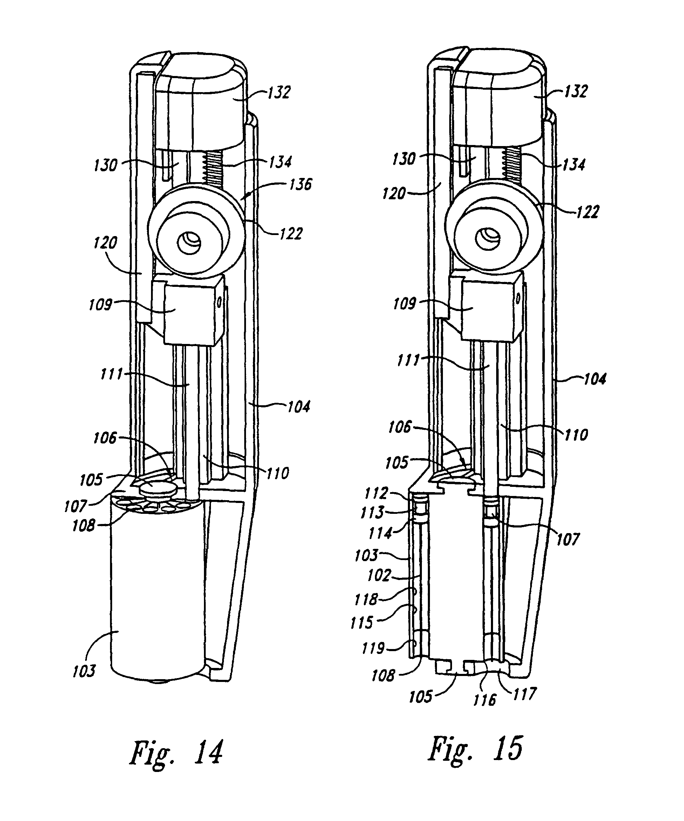

Figure 14 is a partial sectional view of the introducer and angle of insertion assembly of Figure 13.
Figure 15 is a sectional view of the introducer and angle of insertion assembly of Figure 13.

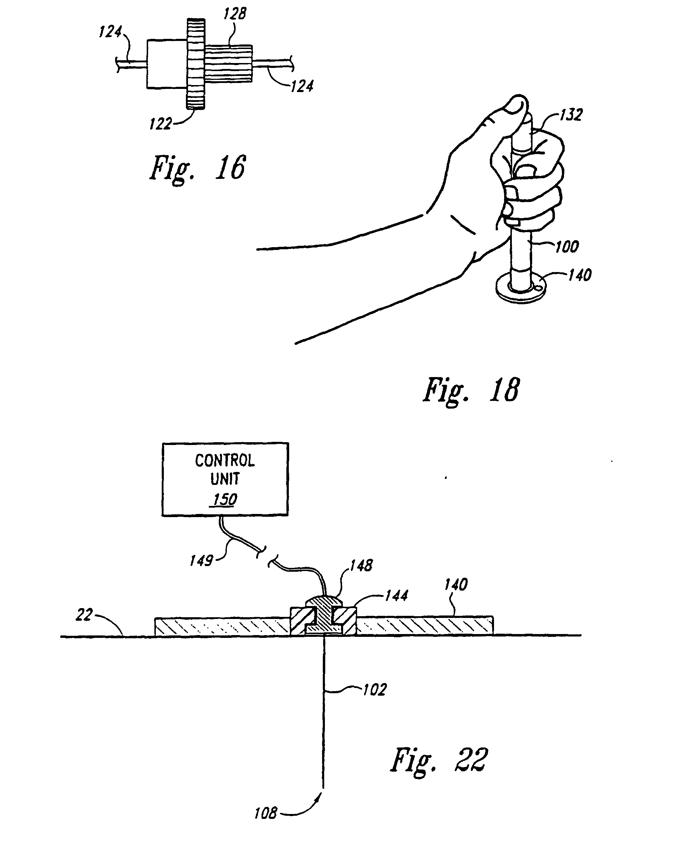

Figure 16 is an elevational view of gear assemblies of the introducer and angle of insertion assembly of Figure 13.

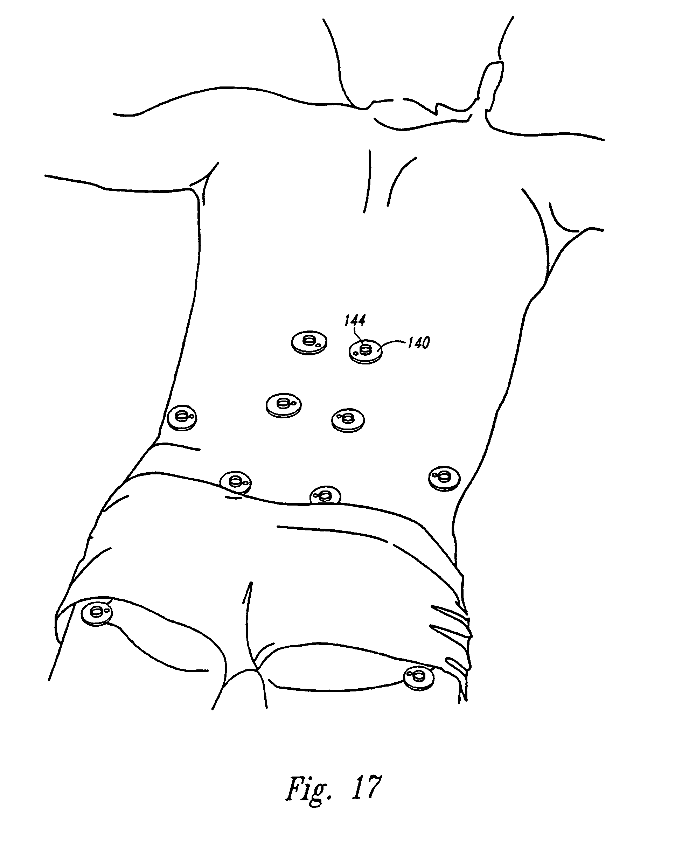

Figure 17 shows part of the electrode assembly of the embodiment of Figures 13-16 in a montage used for treating low back pain using PNT.

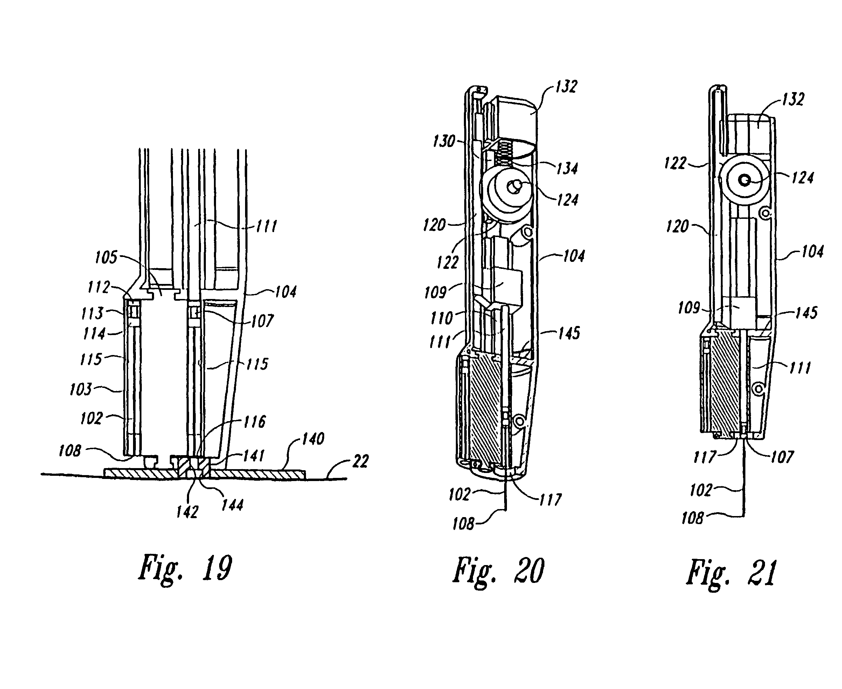

Figure 18 is an elevational view showing the introducer of Figure 13 in the process of deploying an electrode.
Figure 19 is a sectional view showing the introducer of Figure 13 in the process of deploying an electrode, prior to insertion of the electrode.
Figure 20 is a sectional view showing the introducer of Figure 13 in the process of deploying an electrode, during insertion of the electrode.
Figure 21 is a sectional view showing the introducer of Figure 13 in the process of deploying an electrode, also during insertion of the electrode.

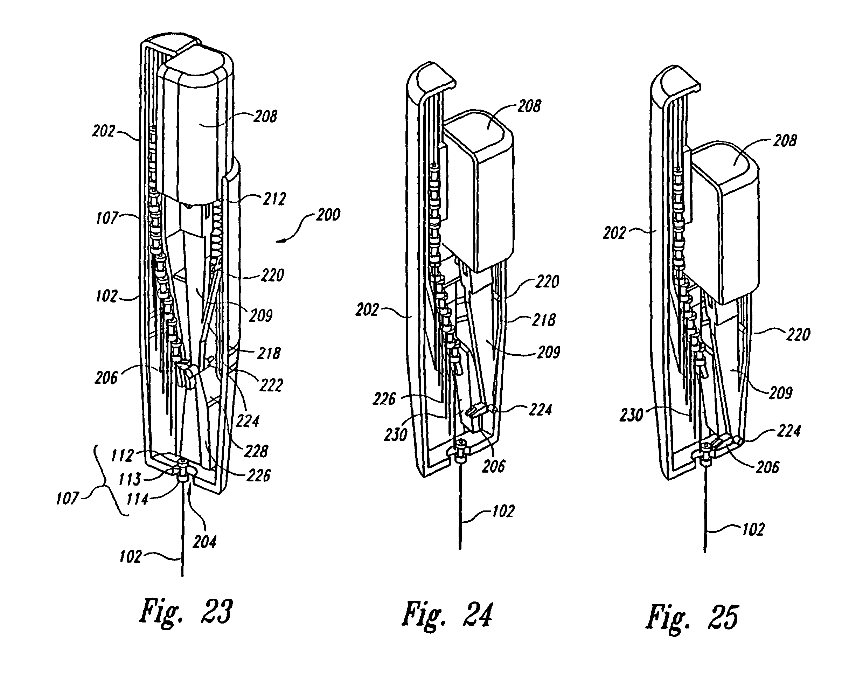

Figure 22 is a sectional view of an inserted electrode assembly of the embodiment of Figures 13-16.
Figure 23 is a partial sectional view of an electrode remover and angle of insertion assembly according to yet another embodiment of the invention prior to removal of an electrode.
Figure 24 is a partial sectional view of the electrode remover and angle of insertion assembly of Figure 23 partially actuated but prior to removal of an electrode.
Figure 25 is a partial sectional view of the electrode remover and angle of insertion assembly of Figure 23 partially actuated but prior to removal of an electrode.

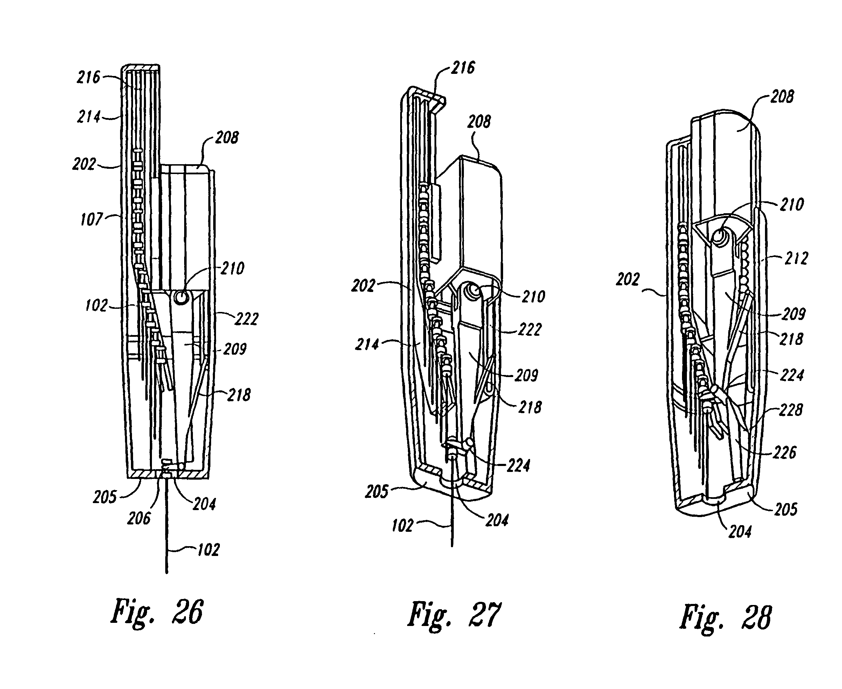

Figure 26 is a partial sectional view of the electrode remover and angle of insertion assembly of Figure 23 partially actuated and engaged with an electrode but prior to removal of the electrode.
Figure 27 is a partial sectional view of the electrode remover and angle of insertion assembly of Figure 23 during removal of an electrode.
Figure 28 is a partial sectional view of the electrode remover and angle of insertion assembly of Figure 23 after removal of an electrode.

### DETAILED DESCRIPTION OF THE PREFERRED EMBODIMENTS

Percutaneous electrical therapy systems, such as PNT systems, deliver electric current to a region of a patient's tissue through electrodes that pierce the skin covering the tissue. The electric current is generated by a control unit external to the patient and typically has particular waveform characteristics such as frequency, amplitude and pulse width. Depending on the treatment or therapy being delivered, there may be one electrode containing both a cathode and an anode or a plurality of electrodes with at least one serving as a cathode and at least one serving as an anode.

The electrode has a sharp point not only to facilitate insertion through the patient's skin but also to enhance local current density during treatment. The placement and location of the electrode's point is therefore an important aspect of the therapy. The angle at which the electrode enters the patient's tissue helps determine where the electrode's point will end up. One aspect of the invention therefore provides an electrode angle of entry assembly for use with a percutaneous electrical therapy system.

Insertion of percutaneous electrodes can be painful. The thinner the electrodes, however, the less pain on insertion. One drawback of conventional thin percutaneous electrodes is they may bend or buckle if they are inserted into the patient improperly. In addition to potentially causing pain to the patient, the sharp point of a bent or buckled electrode will not likely be positioned at the target location for providing the therapy. Since the sharp point of the electrode enhances local current density during treatment, a displaced point could adversely affect the efficacy of the treatment. Another aspect of this invention therefore provides an axial electrode insertion supporter for a percutaneous electrical therapy system. Furthermore, patient apprehension of imagined or impending pain can cause discomfort. Therefore, yet another aspect of this invention provides an electrode insertion pain reducer for use with a percutaneous electrical therapy system and provides other features for minimizing patient discomfort.

Once the electrode is placed in the patient, it is important that the electrode remain stationary so it does not move back out or become completely dislodged. Accordingly, another aspect of this invention provides an inserted electrode holding mechanism for use with a percutaneous electrical therapy system. Furthermore, once the electrode is inserted into the skin, the sharp point may become exposed to pathogens, microbes, toxins, *etc*. in the patient's tissue and/or blood. After removal of the electrode from the patient's tissue, a caregiver or other bystander may be stuck accidentally with the sharp point of the electrode, thereby exposing the caregiver to any pathogens that may be on the used electrode. Another aspect of this invention therefore provides an electrode assembly and/or remover for a percutaneous electrical therapy system that provides sharp point protection and is easy to use.

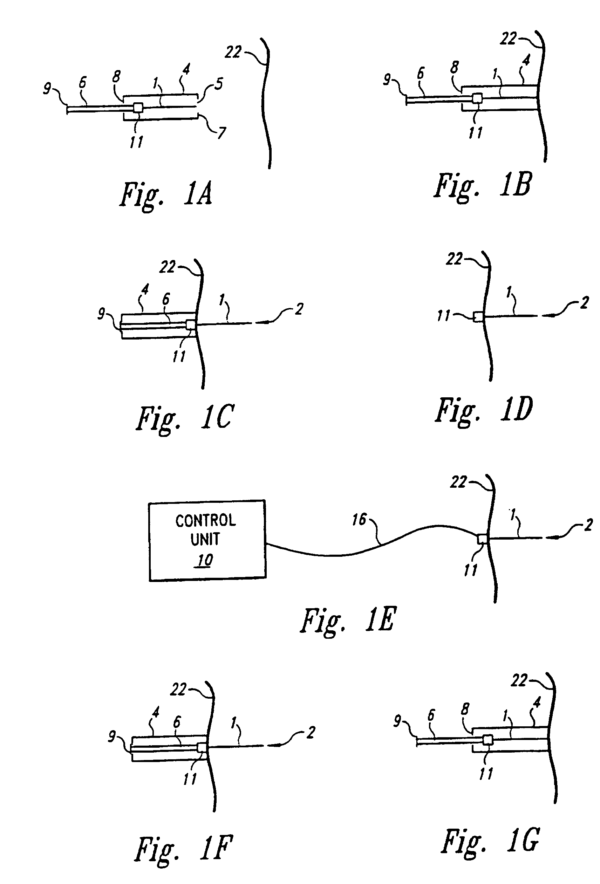

Figures 1A-G are block diagrams showing deployment and use of one embodiment of this percutaneous electrical therapy system and electrode assembly invention. As shown in Figures 1A and 1B, the system includes an electrode 1 having a sharp point 2 at its distal end and a housing 4 surrounding at least the electrode's sharp point 2 when the electrode is in its undeployed and uninserted states. The undeployed and uninserted states include pre-deployment and post-deployment states of the electrode. Housing 4 has an aperture 5 at its distal end. An actuator 6 interacts with a handle 11 at the proximal end of electrode 2 as shown.

Deployment of the electrode assembly includes the steps taken to place the electrode assembly in proper position and condition for use in electrical therapy. Figure 1A shows the electrode assembly in an undeployed (pre-deployed) state. During deployment, the distal face 7 of housing 4 is placed against a patient's skin 22, as shown in Figure 1B. This action supports housing 4 with respect to the patient's skin, thereby controlling the angle between the housing and the patient's skin. Electrode 2 is then inserted through aperture 5 into the tissue underlying the patient's skin by moving actuator 6 distally, as shown in Figure 1C. As it moves, actuator 6―and therefore electrode 2―is supported by housing 4 to control the electrode's angle of entry into the patient's tissue.

Actuator 6 may have a limit stop 9 element cooperating with a limit stop area 8 of housing 4 to limit distal motion of actuator 6 and to control the depth of insertion of sharp point 2 of electrode 1. In a preferred embodiment of the invention, for example, where the electrical therapy system is used to provide percutaneous neuromodulation therapy, the predetermined electrode depth is 3 cm. Other electrode depths may be used, of course, depending on the intended application and therapy.

After insertion, housing 4 and actuator 6 (which have heretofore acted as an electrode introducer) are preferably removed, as shown in Figure 1D. Electrode 1 is connected to a control unit 10 via a conductor or cable 16. For use with PNT, control unit 10 preferably supplies a current-regulated and current-balanced waveform with an amplitude of up to approximately 20 mA, frequency between approximately 4 Hz and 50 Hz, and pulse width of between approximately 50 µsec and 1 msec. Other electrical waveforms having other parameters may be used, of course, depending on the therapy to be provided. Also, while Figure 1E shows only one electrode connected to the control unit, it should be understood that a plurality of electrodes may be connected to a single control unit, as called for by the desired electrical stimulation treatment.

After completion of the electrical therapy, the electrode assembly is undeployed. The patient, therefore, does not have an opportunity to view the length or amount of the electrode that had been inserted into his or her tissue. One embodiment of this invention therefore minimizes any discomfort the patient may experience due to fear or apprehension regarding percutaneous electrodes. In this embodiment, as shown in Figure 1F, the aperture 5 of housing 4 is placed over the handle portion 11 of electrode 1. Housing 4 may be the same used to deploy and insert the electrode (*i*.*e*., the electrode introducer), or it may be an entirely different assembly (*e*.*g*., an electrode remover). The sharp point 2 of electrode 1 is then drawn into housing 4 of sharp point protection assembly 3 by moving actuator 6 proximally, as shown in Figure 1G. Thus, sharp point protection assembly 3 of Figures 1A-G helps prevent all unintended contact between the electrode's sharp point and a caregiver or other bystander before, during and after deployment of the electrode.

Figures 2A-E are block diagrams showing another embodiment of our invention. A control unit 10 is connected to an electrode 12 within an electrode assembly 13 via a conductor 16. As above, for use with PNT, control unit 10 preferably supplies a current-regulated and current-balanced waveform with an amplitude of up to approximately 20 mA, frequency between approximately 4 Hz and 50 Hz, and pulse width of between approximately 50 µsec and 1 msec. Other electrical waveforms having other parameters may be used, of course, depending on the therapy to be provided. Also, while Figures 1 A-E show only one electrode connected to the control unit, it should be understood that a plurality of electrodes may be connected to a single control unit, as called for by the desired electrical stimulation treatment.

As shown in its undeployed state in Figure 2A and in its uninserted state in Figure 2B, the system includes a housing 18 surrounding the sharp point 20 of electrode 12 when the electrode point 20 has not yet been inserted through the patient's skin 22. To begin deployment, distal face 21 of housing 18 is placed against the patient's skin 22, as shown in Figure 2B. As with the previous embodiment, this action supports housing 18 with respect to the patient's skin, thereby controlling the angle between the housing and the patient's skin. In one aspect of this embodiment, the housing is held in place with an adhesive. The system includes an electrode actuator 19 that enables deployment and insertion of the sharp point 20 of electrode 12 through the patient's skin 22 into the underlying tissue through an aperture 24 in housing 18, as shown in Figure 1C. Actuator 19 has an interference fit with housing 18. Since the housing 18 is fixed on the patient's skin, the interference fit between the actuator 19 and the housing 18 requires a minimum force to move actuator 19 with respect to housing 18. This interference fit will keep actuator 19―and therefore electrode 12―in place after electrode point 20 has been placed at the desired location. The combination of the housing's attachment to the patient and the actuator's fixed position with respect to the housing constitutes the electrode holding mechanism of this embodiment.

Actuator 19 may be part of the electrode assembly 13 or a separate component of the system. Actuator 19 may also have a limit stop element 23 that cooperates with a limit stop area 17 of housing 18 to limit distal movement of actuator 19, thereby controlling depth of insertion of electrode 12. In one embodiment of the invention, for example, where the electrical stimulation system is used to provide percutaneous neuromodulation therapy, the predetermined electrode depth is approximately 3 cm., although other electrode depths may be used depending on the application. The control unit 10 may then provide the appropriate therapy to the patient through electrode 12 and any other electrodes connected to it.

During undeployment, actuator 19 is used to draw electrode 12 back proximally into housing 18. After removal of the electrode from the patient's skin, housing 18 of sharp point protection assembly 14 once again surrounds the sharp point 20 of the now uninserted electrode 12, as shown in Figures 2D and 2E. Actuator 19 helps enable this operation to occur without ever exposing the sharp point of the electrode when the sharp point is no longer in the patient. In fact, the operator of the electrode assembly never sees the sharp point of the electrode. Thus, sharp point protection assembly 14 shields the potentially contaminated portion of the undeployed electrode and protects the patient's caregiver or other bystander from unintended contact with the sharp point of the electrode before, during and after electrical therapy.

While Figures 2A-E show the electrode connected to the control unit prior to deployment and insertion of the electrode into the patient's skin, the connection between the control unit and the electrode could be made during deployment or after insertion. Also, while Figures 2A-E show only one electrode connected to the control unit, it should be understood that a plurality of electrodes may be connected to a single control unit, as called for by the desired electrical stimulation treatment.

To use the percutaneous electrical therapy systems of Figures 1A-G and Figures 2A-E to treat a patient, one or more electrodes are inserted through the patient's skin into the underlying tissue. As an example, to treat low back pain using PNT with unipolar electrodes, an array or montage such as that shown in Figure 3 may be used. The "T12" 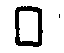 "S1" designations refer to the patient's vertebrae. The control unit or generator supplies current pulses between pairs of electrodes for durations of a few minutes to several hours, preferably delivering the current-regulated waveform described above. Thirty-minute treatments are recommended in the Ghoname *et al*. low back pain treatment articles.

Figures 4-12 show another embodiment of this invention. An electrode assembly 30 includes a base 32, an electrode 34, and a plunger or actuator 36. Base 32 has a flange or flared end 44 that is adapted to make contact with a patient's skin. Base 32 may be formed from any suitable polymer or metal, such as a high-density polyethylene (HDPE). Base 32 is preferably opaque so that the electrode cannot be seen by a needle-shy patient.

Actuator 36 fits within a housing portion 40 of base 32 in a slidable arrangement. A locking assembly is operable to prevent relative movement between actuator 36 and housing 40 of base 32. In this embodiment, the locking assembly of actuator 36 has integrally-formed resilient detents 48 on its exterior cylindrical surface. In the undeployed state of electrode assembly 30, detents 48 mate with a corresponding openings 50 in base 32 to hold actuator 36 and base 32 in place with respect to each other to prevent electrode 34 from moving outside of the protective housing 40 of base 32 and thereby providing sharp point protection. Mechanisms other than the detent and opening arrangement shown here may be used to hold the actuator and base in place may be used without departing from the invention.

In this embodiment, electrode 34 is preferably a 3-cm long 32-gauge stainless steel needle. Other sizes and materials may be used for electrode 34, of course, without departing from the scope of the invention. Actuator 36 is preferably formed from HDPE as well, although other suitable materials may be used.

Electrode 34 has a larger-diameter handle 52 at its proximal end. Handle 52 fits within a channel 54 formed within actuator 36. Channel 54 has a narrow opening 56 at its distal end whose diameter is slightly larger than the diameter of electrode 34 but narrower than the diameter of handle 52 to hold electrode 34 in place within actuator 36 after initial manufacture and assembly. As shown in Figure 7, in an undeployed state the sharp point 38 of electrode 34 is disposed within housing portion 40 of base 32, specifically, within a narrow channel 42 of the housing 40.

To deploy one or more electrode assemblies on a patient in order to provide electrical stimulation therapy (such as PNT), the distal surface 46 of flange portion 44 of base 32 is mounted on the desired site on the patient's skin, preferably with a compressible adhesive pad (not shown) surrounding a ring 43 extending downward from surface 46 around an aperture 41 formed at the distal end of channel 42, although other means of attaching base 32 to the patient may be used as appropriate. This action aligns base 32 with respect to the patient's skin. Flange portion 44 of base 32 provides extra stability for the electrode assembly during electrode insertion and use.

An electrical connector and actuator tool 60 is used to insert the electrode and connect the electrode electrically with a control unit 62. Actuator tool 60 and electrode assembly 30 also interact to provide the sharp point protection assembly of this embodiment. When the distal end of actuator tool 60 is placed against the proximal ends of base 32 and actuator 36, the exposed proximal end 64 of electrode handle 52 makes electrical contact with a contact surface 66 within actuator tool 60. Contact surface 66, in turn, is electrically connected to the control unit 62 via a cable or other conductor 68.

Actuator tool 60 has two oppositely disposed pegs 70 extending outward from the distal portion of its cylindrically surface. Pegs 70 mate with two corresponding slots 72 in actuator 36 and with two corresponding grooves 74 in base 32. (The second slot 72 and second groove 74 are each opposite the slot 72 and groove 74, respectively, shown in Figures 4 and 5.) When connecting actuator tool 60 to electrode assembly 30, pegs 70 move along longitudinal portions 76 of slots 72 and along longitudinal portions 78 of grooves 74. Concurrently, exposed distal end 64 of electrode handle 52 begins to make sliding contact with contact surface 66 of actuator tool 60 to create the electrical connection between actuator tool 60 and electrode 32.

Clockwise rotation (looking down on the assembly) of actuator tool 60 after pegs 70 reach the end of longitudinal portions 76 and 78 moves pegs 70 into short circumferential portions 80 and 82, respectively, of slots 72 and grooves 74. The length of circumferential portions 80 of slots 72 is less than the length of circumferential portions 82 of grooves 74. Continued movement of pegs 70 along circumferential portions 82 will therefore move pegs 70 against the ends 81 of circumferential slots 80. Further clockwise rotation of actuator tool 60 will cause actuator 36 to rotate clockwise as well, thereby moving detents 48 out of openings 50 and allowing the electrode 34 and actuator 36 to move with respect to base 32.

Second longitudinal portions 84 of grooves 74 are formed in base 32 at the end of circumferential portions 82. Movement of pegs 70 distally along longitudinal portions 84 pushes pegs 70 against the distal edges of circumferential slot portions 80, thereby moving actuator 36 and electrode 34 in a controlled fashion distally toward the patient's skin 22.

As it moves, electrode 34 passes through channel 42, and the sharp point of electrode 34 moves out through aperture 41. Channel 42 and actuator 36 provide axial support to electrode 34 during this forward movement and also, along with the support provided by flange 44, provide entry angle guidance to the electrode. In addition, downward pressure on the patient's skin during electrode deployment and/or movement of the actuator tool and actuator compresses the compressible adhesive pad and presses ring 43 against the patient's skin 22, which helps ease electrode entry through the skin and also lessens the insertion pain experienced by the patient.

The alignment of base 32 with respect to the patient's skin and the controlled movement of actuator 36 and electrode 34 within base 32 controls the electrode's angle of entry into the tissue underlying the patient's skin. Distal movement of the electrode and its actuator within base 32 continues until the distal surface 86 of a cylindrical cap portion 92 of actuator tool 60 meets an annular surface 88 of housing 40. At this point, sharp point 38 of electrode 34 has extended a predetermined depth into the tissue underlying the patient's skin. In the preferred embodiment, this predetermined depth is approximately 3 cm., although other electrode depths may be desired depending on the treatment to be performed. In one aspect of this embodiment, an interference fit between the inner surface of channel 42 and the outer surface 55 of channel 54 performs this function.

The interaction of the actuator tool with the actuator and electrode enables the electrode to be inserted into the patient and connected electrically with the control unit in a single motion. From a time and motion standpoint, this design provides increased efficiency through the elimination of a motion (*e*.*g*., separately connecting the electrode to the control unit after inserting the electrode in the patient). This efficiency can save the caregiver a great deal of time when multiplied by, *e*.*g*., ten electrodes per patient and five patients per hour.

Electrical stimulation treatment may begin once the electrodes have been deployed and inserted. Control unit 62 supplies stimulation current to the electrodes, *e.g.,* in the manner described in the Ghoname *et al*. articles. The electrical waveform provided by the control unit depends on the application. For example, in an embodiment of a system providing percutaneous neuromodulation therapy, control unit 62 would preferably provide a current-regulated and current-balanced waveform with an amplitude of up to approximately 20 mA, frequency between approximately 4 Hz and 50 Hz, and pulse width of between approximately 50 µsec and 1 msec.

The interaction of actuator tool 60 and base 32 provides stability to electrode 34 and its electrical connection to the control unit during treatment by holding the electrode in place, by providing strain relief for tugging forces on cable 68, and by providing a robust mechanical connection. It should also be noted that the sharp point of the electrode is not exposed to the operator or to any other bystander at any point during deployment and use of the electrode assembly.

After treatment has been completed, the electrode may be removed from the patient. To do so, actuator tool 60 is moved proximally away from the patient. As pegs 70 move proximally along longitudinal portions 84 of grooves 74, pegs 70 push against proximal edges of the actuator's circumferential slot portions 80, thereby moving actuator 36 and electrode 34 proximally as well. When pegs reach the proximal end of longitudinal groove portions 84, the sharp end 38 of electrode 34 is out of the patient and safely inside housing 40 of base 32. Counterclockwise movement of actuator tool 60 moves pegs along circumferential portions 80 and 82 of slot 72 and groove 74, respectively. Since, as discussed above, circumferential portion 80 is shorter than circumferential portion 82, this counterclockwise movement will turn actuator 36 counterclockwise.

At the limit of the counterclockwise movement, detents 48 move back into openings 50 to prevent further movement of the electrode and actuator with respect to base 32. Further distal movement of actuator tool 60 moves pegs 70 distally along longitudinal portions 76 and 78 of slot 72 and groove 74, respectively, to disconnect actuator tool 60 from electrode assembly 30. Base 32 can then be removed from the patient. It should be noted that the patient never sees the length or amount of the electrode that had been inserted into his or her tissue. One embodiment of this invention can therefore minimize any discomfort the patient may experience due to fear or apprehension regarding percutaneous electrodes.

Once again, the interaction of the actuator tool with the actuator and electrode enables the electrode to be removed from the patient and disconnected electrically from the control unit in a single motion. From a time and motion standpoint, this design provides increased efficiency through the elimination of a motion, particularly when multiplied by many electrodes and many patients. Also, at no time during the electrode deployment, use or removal processes is the sharp point of the electrode exposed to the operator or bystanders.

Figures 10-12 show the use of the electrode and sharp point protection assemblies of Figures 4-9 to treat low back pain using PNT. As shown in Figure 10, ten electrode assemblies 30a-j are arranged in a montage on the patient's back and attached with adhesive. Next, ten actuator tools 60a-j are attached to the ten electrode assemblies 30a-j. In this example, prior to deployment the actuator tools are mounted on an actuator tool tray 61 that provides electrical communication to a control unit 62 via cable 69. The actuator tools electrically connect with tool tray 61, and thereby to cable 69 and control unit 62, via individual cables 68a-j. It should be understood that the tool tray 61 and its electrical connection scheme play no part in this invention. Figure 11 shows the beginning of the electrode insertion process.

Once each electrode assembly has been actuated by its respective actuator tool to insert an electrode into the patient's tissue (as shown in Figure 12), control unit 62 provides electrical signals to treat the patient. Preferably, half the electrodes (*e*.*g*., assemblies 30b, 30d, 30g, 30h and 30i) are treated as anodes, and the other half as cathodes. In the preferred embodiment, control unit 62 would provide a current-regulated and current-balanced waveform with an amplitude of up to approximately 20 mA, frequency between approximately 4 Hz and 50 Hz, and pulse width of between approximately 50 µsec and 1 msec. to treat the patient's low back pain using PNT.

Another embodiment of the invention is shown in Figures 13-28. In this embodiment, an electrode introducer and an alignment member mounted on the patient's skin provide an electrode angle of insertion assembly controlling the electrode's angle of entry into the patient's tissue. The electrode introducer and an electrode remover can cooperate to connect and disconnect an electrode and an electrode holding mechanism, and can provide sharp point protection. In a preferred embodiment of an electrode introducer 100 shown in Figures 13-16 and 19-21, introducer 100 is designed to insert multiple electrodes. It should be understood that the principles of this invention could be applied to an introducer designed to hold and insert any number of electrodes.

Twelve electrodes 102 are disposed within a magazine 103 rotatably mounted within a housing 104. In this embodiment, housing 104 is a two-part injection molded polystyrene assembly. Housing 104 is preferably opaque so that the patient cannot see the length of the electrodes. As seen best in Figure 14, magazine 103 rotates about a hub 105 mounted on supports formed in housing 104. A leaf spring 106 mates with one of twelve radial grooves 108 formed in magazine 103 to form a twelve-position ratchet mechanism for rotatable magazine 103 in housing 104.

Magazine 103 has twelve electrode chambers 115 arranged radially about hub 105. When introducer 100 is completely full, each chamber 115 contains one electrode 102. The diameter of upper portion 118 of chamber 115 is sized to form an interference fit with the wider portions 112 and 114 of electrode handle portion 107 of electrode 102. Lower wide portion 114 of electrode 102 is formed from a compressible material. The diameter of lower portion 119 of chamber 115 is slightly larger so that there is no interference fit between chamber portion 119 and electrode handle 107, for reasons explained below. Each time leaf spring 106 is within a groove 108, the opening 106 of a magazine chamber 115 is lined up with the aperture 117 of introducer 100, as shown in Figures 14 and 15.

A slide member 109 is disposed on a rail 110 formed in housing 104. Extending longitudinally downward from slide member 109 is a drive rod 111, and extending longitudinally upward from slide member 109 is a gear rack 120. The teeth of gear rack 120 cooperate with teeth on a rotational gear 122 mounted about a shaft 124 extending into a shaft mount 126 formed in housing 104. A second set of teeth are mounted on a smaller diameter rotational gear 128 (shown more clearly in Figure 16) which is also mounted about shaft 124. Gears 122 and 128 rotate together about shaft 124.

The teeth of smaller diameter gear 128 mesh with the teeth of a second gear rack 130 extending from a longitudinally movable actuator 132. A spring 134 mounted between actuator 132 and a spring platform 136 biases actuator 132 away from housing 104. Actuator 132, gears 122 and 128, gear racks 120 and 130, slide member 109 and drive rod 111 form the introducer's transmission assembly.

To deploy the electrode assembly of this embodiment, a flexible and compressible annular patch 140 is placed on the patient's skin at the desired site, preferably with adhesive (not shown). For example, to treat low back pain using PNT, the arrangement or montage shown in Figure 17 may be used. In this montage, five electrodes serve as cathodes and five serve as anodes.

As shown in Figures 19 and 20, patch 140 has an annular rigid member 141 disposed in its center and extending upwardly from it. Rigid member 141 has a smaller diameter opening 142 leading to a larger diameter opening 144. The diameter of opening 142 is slightly smaller than the lower wide portion 114 of the handle portion 107 of electrode 102 and slightly larger than the diameter of the central portion 113 of handle portion 107 of electrode 102.

After the patch 140 is in place, the distal end of introducer 100 is placed against patch 140 so that introducer aperture 117 surrounds the upwardly extending portion of rigid patch member 141, as shown in Figure 18. This interaction aligns the opening 116 of one of the introducer's magazine chambers 115 with the opening 142 of rigid member 141 and helps control the electrode's angle of entry, as shown in Figure 19. The line-of-sight action of the introducer (*i*.*e*., the electrode moves along, or parallel to, the introducer's longitudinal axis) helps in the accurate placement of the electrodes.

Downward pressure on introducer 100 compresses patch 140, thereby causing the upper surface of rigid member 141 to engage a lower surface of magazine 103 and pressing rigid member 141 downward into the patient's skin 22. This pressure on the patient's skin around the insertion site minimizes the pain of insertion of the electrode.

Depressing actuator 132 moves gear rack 130 distally, which causes gears 128 and 122 to rotate. Because of the relative diameters and relative tooth counts of gears 128 and 122, gear rack 120 moves longitudinally a much greater distance than the corresponding longitudinal movement of gear rack 130. This feature enables the electrode to be inserted its required distance into the patient's skin using only a comparatively small movement of the operator's thumb and (along with the opaque introducer housing) helps minimize discomfort caused by patient fear and apprehension regarding the length of the electrode being inserted into his or her tissue. Distal movement of gear rack 120 is guided by the movement of slide member 109 along rail 110. As slide member 109 moves distally, drive rod 111 moves into a magazine chamber 115 until the distal end of drive rod 111 engages the top surface of the electrode's handle portion 107. As shown in Figure 20, further distal movement of drive rod 111 pushes electrode 102 downward so that sharp point 108 of electrode 102 leaves the introducer housing and enters the patient's skin 22 and the tissue beneath the skin. Chamber 115 provides axial stability to the electrode 102 during insertion.

When the top portion 112 of electrode handle portion 107 leaves the smaller diameter portion 118 of magazine chamber 115, it enters the larger diameter portion 119 of chamber 115. At this point (shown in Figure 21), because the diameter of chamber portion 119 is wider than the diameter of the electrode handle 107, the electrode is no longer attached to introducer 100.

Continued downward movement of actuator 132 and drive rod 111 pushes the lower larger diameter portion 114 of electrode handle 107 through the smaller diameter portion 142 of rigid member 141 by compressing handle portion 114. Further downward movement pushes handle portion 114 into the larger diameter portion 144 of rigid member 141 so that the rigid member's smaller diameter portion lies between the larger diameter portions 112 and 114 of the electrode handle 107. This interaction holds the electrode in place in the patient's tissue and helps provide depth control for electrode insertion. In this embodiment, the preferred depth of the electrode's sharp point 108 is approximately 3 cm., although other electrode depths may be desired depending on the treatment to be performed. Slider member 109 also acts as a limit stop at this point when it engages the limit stop area 145 of housing 104, thereby also controlling electrode insertion depth.

In one embodiment, actuator 132 and electrode 102 move in the same direction during insertion: along, or parallel to, the longitudinal axis of the introducer. This common directional movement, along with the ergonomic design of the introducer allowing it to be held and operated by one hand, helps control electrode insertion speed and pressure on the patient.

Magazine 103 is rotated to a new insertion position and placed against an empty patch 140 after insertion of each electrode until all electrodes have been deployed and inserted. A suitable electrical connector 148 such as an alligator clip is electrically connected to electrode 102 through an aperture (not shown) formed in the upper larger diameter portion 112 of electrode handle 107 to provide electrical communication between a control unit 150 and electrode 102 via a cable or other conductor 149, as shown in Figure 22. Patch 140 provides strain relief for electrode 102 by preventing tugging forces on cable 149 from dislodging the electrode from the patient, thereby helping keep the electrode in place.

Control unit 150 supplies stimulation current to the electrodes, *e.g.,* in the manner described in the Ghoname *et al*. articles. Once again, the electrical waveform provided by the control unit depends on the application. For example, in an embodiment of a system providing percutaneous neuromodulation therapy, control unit 150 would preferably provide a current-regulated and current-balanced waveform with an amplitude of up to approximately 20 mA, frequency between approximately 4 Hz and 50 Hz, and pulse width of between approximately 50 µsec and 1 msec.

It should be noted that in one embodiment, at no time during the electrode deployment, insertion and electrical therapy treatment processes was the sharp point of the electrode exposed to the operator or bystanders.

In an alternative embodiment, the lower wide portion of the electrode handle is formed from a rigid material and has rounded camming edges. The central annulus of patch 140 in this alternative embodiment is either compressible or has a resilient camming opening under the camming action of the electrode handle.

Figures 23-28 show a sharps-safe electrode remover according to one embodiment of this invention. Remover 200 is designed to work with the electrode and electrode patch assembly described with respect to Figures 13-22 above. It should be understood that the principles of sharps-safe remover 200 may apply to other electrode designs as well.

Remover 200 has a housing 202 with an aperture 204 at its distal end. A number of previously deployed electrodes 102 are stored within housing 202. Housing 202 can be opaque so that the patient cannot see the length of the electrodes being removed. This feature helps minimize discomfort caused by patient fear and apprehension regarding the length of inserted electrodes. A pair of rails 214 and 216 hold the electrodes 102 in alignment via the electrode handles 107, as shown. While this embodiment of the remover is designed to provide sharps-safe removal and storage of a plurality of electrodes, the invention applies to removers designed to remove and store one or any number of electrodes.

As described above, electrodes for percutaneous electrical therapy are inserted through a patient's skin into underlying tissue with handle portions exposed above the skin. The first step in undeploying and removing an inserted electrode is to line up the exposed handle 107 of an electrode with the remover's aperture 204, as shown in Figure 23, by placing the distal face 205 of remover 200 against the patient's skin or against any portion of the electrode assembly (such as an adhesive patch) surrounding the electrode. While not shown in Figures 23-28, aperture 204 is sized to surround an annular member (such as annular member 141 discussed above) holding an electrode handle of an electrode assembly (such as that shown in Figures 13-22 above), the sharp point of which has been inserted through a patient's skin.

An electrode engagement fork 206 is pivotably attached to a longitudinally movable actuator 208 via an arm 209 and a hinged pivot 210. A coil spring 212 biases actuator 208 upward towards the actuator and fork position shown in Figure 28. A leaf spring 218 extends from arm 209. A cross-bar 220 at the end of leaf spring 218 slides in groove 222 and a corresponding groove (not shown) on the other side of housing 202. Leaf spring 218 is in its relaxed state in the position shown in Figure 23. In this position, a cross-bar 224 extending from the distal end of arm 209 adjacent fork 206 lies at the top of a camming member 226 and a corresponding camming member (not shown) on the other side of housing 202.

Downward movement of actuator 208 (in response, *e.g.*, to pressure from a user's thumb) against the upward force of spring 212 moves cross-bar 224 against a first camming surface 228 of camming member 226, as shown in Figure 24. Camming surface 228 pushes crossbar 224 of arm 209 against the action of leaf spring 218 as actuator 208, arm 209 and fork 206 move downward.

Figure 25 shows the limit of the downward movement of fork 206. At this point, crossbar 224 clears the camming member 226, and leaf spring 218 rotates fork 206 and arm 209 about pivot 210 to engage fork 206 with electrode handle 107, as shown in Figure 26. The tine spacing of fork 206 is shorter than the diameter of the upper wide portion 112 of electrode handle 107 but wider than the diameter of the narrow middle portion 113 of electrode handle 107.

Release of actuator 208 by the user permits spring 212 to move actuator 208, arm 209 and fork 206 proximally. The engagement between fork 206 and electrode handle 107 causes the electrode to begin to move proximally with the fork out of the patient and into the remover housing, as shown in Figure 27. At this point, crossbar 224 is now engaged with a second camming surface 230 of camming member 226. Camming surface 230 pushes cross-bar 224 against the action of leaf spring 218 in the other direction (to the left in the view shown in Figure 27) as the electrode, fork and arm rise under the action of coil spring 212.

The electrode and fork continue to rise until they reach the upward limit of their permitted motion, as shown in Figure 28. At this point, electrode handle 107 has engaged rails 214 and 216 and the most recent electrode previously stored in remover 200. Electrode handle 107 pushes against the electrode handle of the previously stored electrode handle, which in turn pushes against any electrode handles stored above it in the stack. In this manner, the latest electrode removed by remover 200 goes into the bottom of the stack of used electrodes stored in remover 200. Now that the sharp point 108 of electrode 102 is safely inside housing 202, remover 200 can be withdrawn from the site on the patient's skin through which the electrode had been inserted. Once cross-bar 224 clears the top of camming member 226, and leaf spring 218 moves arm 209 back to the center position shown in Figure 23.

It should be noted that the remover 200 can provide sharp point protection for the entire electrode undeployment and removal process. Once all electrodes have been removed, the used electrodes can be safely transported in the sharps-safe container provided by the housing 202 of remover 200.

The method for administering percutaneous electrical therapy to a patient, comprising the use of the apparatus of the invention comprises:
aligning an electrode housing with tissue of the patient;
moving at least one of the electrode and the housing relative to the other to insert a sharp point of the electrode into the tissue;
controlling a motion of and/or access to the electrode with the housing; and
applying an electrical current to the housing while the electrode is inserted in the tissue.

Preferably the aligning an electrode housing includes placing a distal face of the housing against the tissue of the patient, or
aligning an electrode housing includes attaching a distal face of the electrode housing to the tissue of the patient.

Preferably the method further comprises supporting the electrode relative to the housing while moving the electrode relative to the housing along an axis approximately perpendicular to the tissue.

Preferably the method comprises guiding the electrode in an axial direction by engaging at least a portion of the electrode with walls of a channel disposed annularly about the electrode as the electrode moves relative to the housing.

Preferably the moving at least one of the electrode and the housing includes operatively coupling an actuator with the electrode and moving the actuator a predetermined actuator distance, the actuator moving the electrode to insert the sharp point of the electrode into the tissue, wherein preferably further the following steps are carried out:
moving the electrode a selected depth into the tissue; and
simultaneously moving the actuator by the actuator distance with the actuator distance less than the selected depth.

Preferably the controlling motion of the electrode includes halting movement of the electrode when the sharp point of the electrode reaches a selected depth in the tissue.

Preferably the moving at least one of the electrode and the housing includes engaging an actuator with the electrode and moving the actuator relative to the housing, and wherein controlling motion of the electrode includes engaging the actuator with a limit stop of the housing.

Preferably the method further comprises releasing the electrode from the housing when the electrode is in the tissue, or
further comprises mounting an electrode holder on the patient and mechanically interacting the electrode with the electrode holder to support the electrode relative to the patient.

This step of mechanically interacting the electrode includes a step comprises inserting the electrode through an opening in the electrode holder.

Preferably the method further comprises engaging an actuator with the electrode, releasably engaging an actuator tool with the actuator, and moving the actuator tool relative to the housing to move the electrode into the tissue, wherein preferably the following step is carried out :
moving the actuator tool relative to the housing to remove the sharp point of the electrode from the patient.

Preferably the method comprises electrically coupling the electrode to an electrode control unit to provide electrical current to the electrode.

Preferably the method comprises placing a patch on the patient, inserting the electrode through an aperture of the patch into the tissue, and supporting the electrode relative to the tissue by engaging the electrode with the patch.

Preferably the method comprises moving at least one of the electrode and the housing includes releasably engaging the electrode with an actuator, moving the actuator relative to the housing, and mechanically engaging the actuator with the housing to hold the electrode in place in the patient.

Preferably the housing supports a plurality of electrodes and wherein the method further comprises successively engaging each electrode with an actuator and moving the actuator relative to the housing to insert each electrode into the tissue.

Preferably the housing includes an actuator coupled to the electrode and wherein the method further comprises engaging the actuator with a human thumb and moving the thumb relative to the housing to move the electrode into the tissue.

Preferably the method further comprises applying pressure to a skin of the patient adjacent to an electrode insertion point as the electrode is passed into the patient at the electrode insertion point.

Preferably the moving at least one of the electrode and the housing includes moving toward the patient an actuator operatively coupled to the electrode to apply pressure to the patient adjacent to a point at which the electrode enters the patient.

Preferably the method further comprises moving at least part of the electrode out of the housing and into the patient and connecting the electrode electrically with a control unit cable with a single motion.

Preferably the method further comprises moving the electrode back into the housing and disconnecting the electrode electrically from a control unit cable with a single user motion.

Preferably the method further comprises inserting the sharp point of the electrode into a patient's tissue without exposing the sharp point to anyone but the patient.

Preferably the method further comprises removing the electrode from the patient's tissue without exposing the sharp point to anyone but the patient.

Preferably the method further comprises
moving the entire electrode out of the housing; and
removing the housing from the patient.

Preferably the housing is a first housing and wherein the method further comprises removing the electrode from the tissue and into a second housing without exposing the sharp point to anyone but the patient.

The percutaneous electrode can be removed as follows:
grasping a housing of a percutaneous electrode remover;
engaging the housing with tissue proximate to the percutaneous electrode while the percutaneous electrode is inserted in the tissue;
manipulating an actuator of the percutaneous electrode remover to couple the actuator to the percutaneous electrode; and
activating the actuator to withdraw the percutaneous electrode from the tissue and into the housing.

Preferably the manipulating the actuator includes applying pressure to the actuator to move the actuator relative to the housing from a second position to a first position and activating the actuator includes reducing pressure applied to the actuator while a spring biases the actuator from the first position to the second position.

Preferably the manipulating the actuator includes engaging the actuator with a human thumb and depressing the actuator relative to the housing.

Preferably the coupling the actuator to the percutaneous electrode includes engaging spaced-apart tine portions with an exposed portion of the percutaneous electrode.

Preferably the removal further comprises simultaneously storing a plurality of removed electrodes in the housing.

Preferably the manipulating the actuator includes depressing the actuator relative to the housing, moving an arm pivotably coupled to the actuator along a first surface of a camming member from an aligned position with the arm aligned with the electrode to a first unaligned position with the arm offset from the electrode, and wherein coupling the actuator to the percutaneous electrode includes disengaging the arm from the first surface of the camming member and biasing the arm to the aligned position, and further wherein activating the actuator includes releasing the actuator while a spring biases the arm along a second surface of the camming member to a second unaligned position offset from the aligned position.

Modifications of the above embodiments of the invention will be apparent to those skilled in the art. For example, while the invention was described in the context of percutaneous electrical therapy in which electrodes are used to deliver electricity to a patient, the entry angle control features may be used with electrodes designed for medical monitoring and/or diagnosis. In addition, the entry angle control features of this invention may be used with acupuncture needles or other needles.

## Claims

1. An apparatus for administering percutaneous electrical therapy, comprising:
an electrode (1, 12, 34) electrically connectable to a control unit to deliver electrical therapy to a patient during operation, the electrode having a first end and a second end opposite the first end, the first end having a sharp point (2, 20, 38) configured to be inserted into tissue of the patient;
an actuator coupled to the electrode to carry the electrode; and
an electrode housing (4, 18, 40, 36) operatively coupled to the actuator with the actuator being received in the electrode housing, the electrode housing being positioned to support the actuator during insertion of the electrode into the tissue, the housing carrying the actuator to guide the actuator and the electrode along a path and/or control access to the electrode, the actuator being movable relative to the electrode housing between a first position with the electrode positioned at least partially within the housing and a second position with the electrode positioned at least partially external to the housing.

2. The apparatus of claim 1, further comprising the control unit, the control unit being coupled to the electrode, and wherein the control unit is configured to direct to the electrode a current-regulated and current-balanced waveform with an amplitude of up to approximately 20 mA and a frequency of from about 4 Hz to about 50 Hz.

3. The apparatus of claim 1 wherein the housing has a distal face configured to be placed on the patient to support the electrode relative to the patient and control an angle between the housing and the patient, the housing having a first diameter at the distal face and a second diameter spaced apart from the distal face, the first diameter being larger than the second diameter.

4. The apparatus of claim I wherein the housing has a distal face configured to be placed on the patient to support the electrode relative to the patient, and wherein the distal face has an aperture (5, 24, 41) configured to surround at least a portion of the electrode during insertion of the electrode into the tissue.

5. An apparatus according to claim 1, further comprising
a mechanial connection between the electrode and the housing during insertion of the electrode into the tissue, the mechanical connection including a peg (70) operatively coupled to the electrode and slidably positioned in a groove (74) of the housing, the groove being positioned parallel to the path along which the electrode is guided, the path including a straight-line axis at a fixed angle relative to the patient when the housing is engaged with the patient.

6. The apparatus of claim 1 wherein the electrode and the housing are configured to remain mechanically connected during application of electrical therapy to the patient.

7. The apparatus of claim 1 wherein the housing is configured to be separated from the electrode after insertion of the electrode into the tissue but before application of electrical therapy to the patient.

8. The apparatus of claim 1, further comprising a housing alignment member (140) disposed on the tissue and adapted to mechanically interact with the housing to align the housing with respect to the tissue.

9. The apparatus of claim 1, further comprising a housing alignment member disposed on the tissue and adapted to mechanically interact with the housing to align the housing with respect to the tissue, the housing alignment member including a patch attached to the tissue.

10. The apparatus of claim 9 wherein the patch includes a compressible portion having an adhesive to attach to the patient, the patch further having a generally rigid annular member positioned to releasably engage the housing and at least partially surround the electrode during insertion of the electrode into the tissue.

11. The apparatus of claim 10 wherein the electrode includes first and second spaced apart, outwardly extending flanges toward the second end, and wherein the rigid annular member includes an inwardly extending lip positioned to fit between the first and second flanges when the electrode is inserted into the tissue by a selected distance.

12. The apparatus of claim 1, further comprising an electrode actuator (12) movable within the housing, the electrode actuator being positioned to move the sharp point of the electrode into the tissue of the patient.

13. The apparatus of claim 1 wherein the housing includes a channel (42) disposed annularly about the electrode and positioned to engage and guide at least a portion of the electrode during operation as the electrode moves relative to the housing into the tissue.

14. The apparatus of claim 1, further comprising a limit stop depending from the housing and positioned to releasably mechanically couple to the electrode and limit a length of travel of the electrode as the electrode moves relative to the housing.

15. The apparatus of claim 1, further comprising:
an electrode actuator attached to the electrode and movable with the electrode relative to the housing; and
a limit stop depending from the housing and positioned to engage the electrode actuator and stop motion of the electrode actuator when the electrode reaches a selected depth in the tissue.

16. The apparatus of claim 1, further comprising:
an electrode actuator attached to the electrode and movable with the electrode relative to the housing; and
an actuator tool removably attached to the actuator to move the actuator and the electrode relative to the tissue.

17. The apparatus of claim 16 wherein the actuator tool has an electrical contact positioned to removably engage the electrode and make electrical communication between the electrode and the control unit.

18. The apparatus of claim 1, further comprising:
a slide member slidably positioned within the housing and slidable relative to the housing to engage the electrode, insert the electrode into the patient and release the electrode; and
a limit stop depending from the housing and positioned to engage the slide member when the electrode reaches a selected depth in the tissue.

19. The apparatus of claim 18, further comprising an actuator movably coupled to the housing and coupled to the slide member with a transmission assembly positioned to insert the electrode a predetermined depth into the tissue when the actuator is moved a predetermined actuator distance, the predetermined depth being greater than the predetermined actuator distance.

20. The apparatus of claim 19 wherein the transmission assembly includes a first rack operatively coupled to the actuator, a second rack operatively coupled to the slide member and a gear assembly positioned between the first and second racks and rotatable about a rotation axis, the gear assembly having first teeth engaged with the first rack and positioned a first diameter from the rotation axis, the gear assembly having second teeth concentric with the first teeth, engaged with the second rack and positioned a second diameter from the rotation axis, the second diameter being greater than the first diameter to transmit a first linear movement of the actuator into a second linear movement of the electrode, the second movement being greater than the first movement.

21. The apparatus of claim 1, further comprising a deployed electrode holding mechanism configured to hold the electrode in place after insertion of the sharp point of the electrode into the patient's tissue.

22. The apparatus of claim 1 wherein the electrode includes an electrode handle positioned to extend exterior to the tissue after insertion of the sharp point into the tissue.

23. The system of claim 1 wherein the housing is configured to be removable attached to the patient.

24. The system of claim 1, further comprising a conductor configured to connect between the electrode and the control unit, wherein the housing is configured to support the conductor and provide strain relief to the electrode.

25. The apparatus of claim 1 wherein the electrode includes an electrical connector portion toward the second end, with the electrode connector portion of the electrode being exposed above skin of the patient when the sharp point of the electrode is in the tissue.

26. The apparatus of claim 1, further comprising an actuator configured to engage a human thumb to move the actuator and the electrode relative to the housing.

27. The apparatus of claim 1 wherein the housing is configured to support a plurality of electrodes, and wherein the apparatus further comprises an actuator moveable relative to the housing to successively engage and move each of the plurality of electrodes individually outwardly from the housing to place the sharp point of each electrode beneath in the tissue.

28. The apparatus of claim 27, further comprising a magazine in the housing and supporting the plurality of electrodes.

29. The apparatus of claim 1, further comprising an actuator releasably coupled to the electrode, with the actuator and the electrode configured to move in the same direction during placement of the sharp point of the electrode in the tissue.

30. The apparatus of claim 1 wherein the housing includes a pressure element positioned adjacent to the electrode to provide pressure against the tissue adjacent to an electrode insertion point through which the electrode enters the tissue.

31. The apparatus of claim 30 wherein the pressure element extends completely around the insertion point during insertion of the electrode.

32. The apparatus of claim 1 wherein the housing includes a distal face positioned to engage a skin of the patient, and wherein the housing includes a pressure element extending beyond the distal face to provide pressure on the skin, the pressure element having an aperture to receive the electrode as the electrode passes into the tissue.

33. The apparatus of claim 32 wherein the pressure element has a first diameter and the distal face has a second diameter greater than the first diameter.

34. The apparatus of claim I wherein the housing is opaque.

35. The apparatus of claim 1 wherein housing is positioned to contain at least the sharp point of the electrode when the electrode is in an undeployed state.

36. The apparatus of claim 1 wherein the sharp point of the electrode is positioned outside of the housing in a deployed state and further wherein a portion of the electrode is positioned within the housing when the electrode is in the deployed state.

37. The apparatus of claim 1 wherein the electrode is moveable relative to the housing between a deployed state and an undeployed state, and further wherein the housing is positioned to contain at least the sharp point of the electrode after the electrode has moved from the deployed state to an undeployed state.

38. The apparatus of claim 1 wherein the housing forms an interface with skin of the patient when the housing is engaged with the skin, the housing and the skin completely surrounding the sharp point of the electrode as the electrode moves into the tissue.

39. The apparatus of claim 1 wherein the housing further includes a locking assembly positioned to prevent relative movement between the electrode and the housing when the locking assembly is engaged.

40. The apparatus of claim 39 wherein the locking assembly includes a spring-biased detent operatively coupled to the electrode and engageable with a corresponding aperture in the housing.

41. The apparatus of claim 39 wherein the locking assembly includes an actuator coupled to the electrode and having a detent releasably engageable with an aperture in the housing, and wherein the apparatus further includes a tool adapted to release the locking assembly and to move the sharp point of the electrode out of the housing.

42. The apparatus of claim 41 wherein the tool is configured to move the sharp point of the electrode back into the housing after having moved the sharp point of the electrode out of the housing and to engage the locking assembly to prevent further relative movement between the electrode and the housing.

43. The apparatus of claim 41 wherein the actuator tool has an electrical contact positioned to make electrical communication between the electrode and a control unit.

44. The apparatus of claim 1 wherein the housing is adapted to contain none of the electrode when the electrode is in a deployed state in which the sharp point of the electrode has been inserted into the tissue.

45. The apparatus of claim 1 wherein the housing is configured to contain a plurality of electrodes and wherein the apparatus further comprises an actuator moveable relative to the housing to move each electrode out of the housing one at a time.

46. The apparatus of claim 1 wherein the housing is a first housing and wherein the apparatus further comprises a second housing to contain at least the sharp end of the electrode when the electrode has moved from a deployed state to an undeployed state.

47. The apparatus of claim 1, further comprising a deployed electrode holding mechanism configured to support the electrode in place after insertion of the sharp point of the electrode into the tissue.

48. The apparatus of claim 1, further comprising the control unit, the control unit being coupled to the electrode, and wherein the control unit is configured to direct to the electrode a current-regulated and current-balanced waveform.

49. A percutaneous electrode remover comprising:
a housing configured to be held in a human hand, the housing having an aperture at a distal end; and
an actuator configured to be engaged by the human hand, the actuator being moveable relative to the housing between a first position with the actuator coupled to an electrode while the electrode is inserted in a patient, and a second position with the actuator coupled to the electrode and the electrode withdrawn through the aperture and completely into the housing.

50. The remover of claim 49, further comprising an electrode engager connected to the actuator and positioned to engage an exposed portion of an electrode during operation, the electrode engager being moveable with the actuator between the first position and the second position.

51. The remover of claim 49 wherein the actuator is configured to engage a human thumb during movement between the first and second positions.

52. The remover of claim 49 wherein the housing includes a used electrode holder positioned to support a plurality of electrodes that have been moved into the housing by operation of the actuator.

53. The remover of claim 49, further comprising:
an arm pivotably coupled to the actuator and moveable relative to an axis extending through the aperture between an aligned position, a first unaligned position on one side of the axis, and an second unaligned position on another side of the axis;
an arm spring coupled between the arm and the housing to bias the arm to the aligned position;
a camming member proximate to the arm, the camming member having a first camming surface engaged with the arm when the actuator moves from the second position to the first position to move the arm from the aligned position to the first unaligned position, the camming member further having a second camming surface engaged with the arm when the actuator moves from the first position to the second position;
an actuator spring coupled between the actuator and the housing to bias the actuator to the second position; and
an engagement fork connected to the arm and having two tines positioned to fit around the electrode and engage the electrode when the actuator is in the first position.

## Patentansprüche

1. Vorrichtung zum Behandeln mit perkutaner Elektrotherapie, die umfasst:
eine Elektrode (1, 12, 34), die elektrisch mit einer Steuereinheit verbunden werden kann, um einem Patienten in Funktion Elektrotherapie zukommen zu lassen, wobei die Elektrode ein erstes Ende und ein zweites Ende gegenüber dem ersten Ende hat und das erste Ende eine scharfe Spitze (2, 20, 38) hat, die zum Einführen in Gewebe des Patienten geformt ist;
ein Betätigungselement, das mit der Elektrode verbunden ist, um die Elektrode zu halten; und
ein Elektrodengehäuse (4, 18, 40, 36), das funktionell mit dem Betätigungselement verbunden ist, wobei das Betätigungselement in dem Elektrodengehäuse aufgenommen ist und das Elektrodengehäuse so positioniert ist, dass es das Betätigungselement beim Einführen der Elektrode in das Gewebe trägt, wobei das Gehäuse das Betätigungselement hält, um das Betätigungselement und die Elektrode auf einem Weg zu führen, und/oder Zugang zu der Elektrode zu steuern, und das Betätigungselement relativ zu dem Elektrodengehäuse zwischen einer ersten Position, in der die Elektrode wenigstens teilweise innerhalb des Gehäuses positioniert ist, und einer zweiten Position bewegt werden kann, in der die Elektrode wenigstens teilweise außerhalb des Gehäuses positioniert ist.

2. Vorrichtung nach Anspruch 1, die des Weiteren die Steuereinheit umfasst, wobei die Steuereinheit mit der Elektrode gekoppelt ist und die Steuereinheit so ausgeführt ist, dass sie eine stromgeregelte und stromausgeglichene Wellenform mit einer Amplitude von bis zu ungefähr 20 mA und einer Frequenz zwischen ungefähr 4 Hz und ungefähr 50 Hz zu der Elektrode leitet.

3. Vorrichtung nach Anspruch 1, wobei das Gehäuse eine distale Fläche hat, die so ausgeführt ist, dass sie an dem Patienten platziert wird, um die Elektrode relativ zu dem Patienten zu tragen und einen Winkel zwischen dem Gehäuse und dem Patienten zu steuern, wobei das Gehäuse einen ersten Durchmesser an der distalen Fläche und einen zweiten Durchmesser hat, der von der distalen Fläche beabstandet ist, und der erste Durchmesser größer ist als der zweite Durchmesser.

4. Vorrichtung nach Anspruch 1, wobei das Gehäuse eine distale Fläche hat, die so ausgeführt ist, dass sie an dem Patienten platziert wird, um die Elektrode retativ zu dem Patienten zu tragen, und wobei die distale Fläche eine Öffnung (5, 24, 41) hat, die so ausgeführt ist, dass sie wenigstens einen Teil der Elektrode beim Einführen der Elektrode in das Gewebe umgibt.

5. Vorrichtung nach Anspruch 1, die des Weiteren umfasst:
eine mechanische Verbindung zwischen der Elektrode und dem Gehäuse beim Einführen der Elektrode in das Gewebe, wobei die mechanische Verbindung einen Zapfen (70) enthält, der funktionell mit der Elektrode verbunden und verschiebbar in einer Nut (74) des Gehäuses positioniert ist, wobei die Nut parallel zu dem Weg positioniert ist, auf dem die Elektrode geführt wird, und der Weg eine geradlinige Achse in einem unveränderlichen Winkel retativ zu dem Patienten einschließt, wenn das Gehäuse mit dem Patienten in Kontakt ist.

6. Vorrichtung nach Anspruch 1, wobei die Elektrode und das Gehäuse so ausgeführt sind, dass sie bei Durchführung von Elektrotherapie an dem Patienten mechanisch verbunden bleiben.

7. Vorrichtung nach Anspruch 1, wobei das Gehäuse so ausgeführt ist, dass es von der Elektrode nach Einführung der Elektrode in das Gewebe, jedoch vor Durchführung von Elektrotherapie an dem Patienten getrennt wird.

8. Vorrichtung nach Anspruch 1, die des Weiteren ein Gehäuse-Ausrichtelement (140) umfasst, das sich an dem Gewebe befindet und so eingerichtet ist, dass es mechanisch mit dem Gehäuse in Wechselwirkung tritt, um das Gehäuse in Bezug auf das Gewebe auszurichten.

9. Vorrichtung nach Anspruch 1, die des Weiteren ein Gehäuse-Ausrichtelement umfasst, das sich an dem Gewebe befindet und so eingerichtet ist, dass es mit dem Gehäuse in Wechselwirkung tritt, um das Gehäuse in Bezug auf das Gewebe auszurichten, wobei das Gehäuse-Ausrichtelement ein Pflaster enthält, das an dem Gewebe angebracht wird.

10. Vorrichtung nach Anspruch 9, wobei das Pflaster einen zusammendrückbaren Abschnitt mit einem Klebstoff zum Anbringen an dem Patienten enthält und das Pflaster des Weiteren ein im Allgemeinen starres, ringförmiges Element aufweist, das so positioniert ist, dass es lösbar mit dem Gehäuse in Eingriff kommt und die Elektrode beim Einführen der Elektrode in das Gewebe wenigstens teilweise umgibt.

11. Vorrichtung nach Anspruch 10, wobei die Elektrode einen ersten und einen zweiten Flansch enthält, die voneinander beabstandet sind und sich nach außen auf das zweite Ende zu erstrecken, und wobei das ringförmige starre Element eine sich nach innen erstreckende Lippe enthält, die so positioniert ist, dass sie zwischen den ersten und den zweiten Flansch passt, wenn die Elektrode über eine ausgewählte Strecke in das Gewebe eingeführt ist.

12. Vorrichtung nach Anspruch 1, die des Weiteren ein Elektroden-Betätigungselement (12) umfasst, das in dem Gehäuse bewegt werden kann, und das Elektroden-Betätigungselement so positioniert ist, dass es die scharfe Spitze der Elektrode in das Gewebe des Patienten bewegt.

13. Vorrichtung nach Anspruch 1, wobei das Gehäuse einen Kanal (42) enthält, der sich ringförmig um die Elektrode herum befindet und so positioniert ist, dass er in Funktion mit wenigstens einem Teil der Elektrode in Eingriff kommt und ihn führt, wenn sich die Elektrode relativ zu dem Gehäuse in das Gewebe hinein bewegt.

14. Vorrichtung nach Anspruch 1, die des Weiteren einen Grenzanschlag umfasst, der von dem Gehäuse nach unten vorsteht und so positioniert ist, dass er lösbar mechanisch mit der Elektrode gekoppelt ist und einen Bewegungsabschnitt der Elektrode begrenzt, wenn sich die Elektrode relativ zu dem Gehäuse bewegt.

15. Vorrichtung nach Anspruch 1, die des Weiteren umfasst:
ein Elektroden-Betätigungselement, das an der Elektrode angebracht ist und mit der Elektrode relativ zu dem Gehäuse bewegt werden kann; und
einen Grenzanschlag, der von dem Gehäuse nach unten vorsteht und so positioniert ist, dass er mit dem Elektroden-Betätigungselement in Eingriff kommt und Bewegung des Elektroden-Betätigungselementes unterbricht, wenn die Elektrode eine ausgewählte Tiefe in dem Gewebe erreicht.

16. Vorrichtung nach Anspruch 1, die des Weiteren umfasst:
ein Elektroden-Betätigungselement, das an der Elektrode angebracht ist und mit der Elektrode relativ zu dem Gehäuse bewegt werden kann; und
ein Betätigungselement-Instrument, das abnehmbar an dem Betätigungselement angebracht ist, um das Betätigungselement und die Elektrode relativ zu dem Gewebe zu bewegen.

17. Vorrichtung nach Anspruch 16, wobei das Betätigungselement-Instrument einen elektrischen Kontakt hat, der so positioniert ist, dass er entfembar mit der Elektrode in Eingriff kommt und elektrische Verbindung zwischen der Elektrode und der Steuereinheit herstellt.

18. Vorrichtung nach Anspruch 1, die des Weiteren umfasst:
ein Schiebeelement, das verschiebbar in dem Gehäuse positioniert ist und in Bezug auf das Gehäuse verschoben werden kann, um mit der Elektrode in Eingriff zu kommen, die Elektrode in den Patienten einzuführen und die Elektrode freizugeben; und
ein Grenzanschlag, der von dem Gehäuse nach unten vorsteht und so positioniert ist, dass er mit dem Schiebeelement in Eingriff kommt, wenn die Elektrode eine ausgewählte Tiefe in dem Gewebe erreicht.

19. Vorrichtung nach Anspruch 18, die des Weiteren ein Betätigungselement umfasst, das beweglich mit dem Gehäuse gekoppelt und mit dem Schiebeelement gekoppelt ist, wobei eine Umwandlungs-Baugruppe so positioniert ist, dass die Elektrode über eine vorgegebene Tiefe in das Gewebe eingeführt wird, wenn das Betätigungselement um eine vorgegebene Betätigungselement-Strecke bewegt wird und die vorgegebene Tiefer größer ist als die vorgegebene Betätigungselement-Strecke.

20. Vorrichtung nach Anspruch 19, wobei die Umwandlungs-Baugruppe eine erste Zahnstange, die funktionell mit dem Betätigungselement gekoppelt ist, eine zweite Zahnstange, die funktionell mit dem Schiebeelement gekoppelt ist, sowie eine Zahnrad-Baugruppe enthält, die zwischen der ersten sowie der zweiten Zahnstange positioniert ist und um eine Drehachse gedreht werden kann, wobei die Zahnrad-Baugruppe erste Zähne hat, die mit der ersten Zahnstange in Eingriff und an einem ersten Durchmesser von der Drehachse positioniert sind, und die Zahnrad-Baugruppe zweite Zähne konzentrisch zu den ersten Zähnen hat, die mit der zweiten Zahnstange in Eingriff und an einem zweiten Durchmesser von der Drehachse positioniert sind, wobei der zweite Durchmesser größer ist als der erste Durchmesser, um eine erste lineare Bewegung des Betätigungselementes in eine zweite lineare Bewegung der Elektrode umzuwandeln, wobei die zweite Bewegung größer ist als die erste Bewegung.

21. Vorrichtung nach Anspruch 1, die des Weiteren einen Haltemechanismus für die platzierte Elektrode umfasst, der so ausgeführt ist, dass er die Elektrode nach dem Einführen der scharfen Spitze der Elektrode in das Gewebe des Patienten festhält.

22. Vorrichtung nach Anspruch 1, wobei die Elektrode einen Elektrodengriff enthält, der so positioniert ist, dass er sich nach dem Einführen der scharfen Spitze in das Gewebe außerhalb des Gewebes erstreckt.

23. System nach Anspruch 1, wobei das Gehäuse so ausgeführt ist, dass es entfernbar an dem Patienten angebracht wird.

24. System nach Anspruch 1, das des Weiteren einen Leiter umfasst, der so ausgeführt ist, dass er die Elektrode und die Steuereinheit verbindet, wobei das Gehäuse so ausgeführt ist, dass es den Leiter trägt und Zugentlastung der Elektrode bewirkt.

25. Vorrichtung nach Anspruch 1, wobei die Elektrode einen elektrischen Verbinderabschnitt in Richtung des zweiten Endes enthält und der Elektroden-Verbinderabschnitt der Elektrode über der Haut des Patienten freiliegt, wenn die scharfe Spitze der Elektrode in dem Gewebe ist.

26. Vorrichtung nach Anspruch 1, die des Weiteren ein Betätigungselement umfasst, das so ausgeführt ist, dass es mit einem menschlichen Daumen in Eingriff kommt, um das Betätigungselement und die Elektrode relativ zu dem Gehäuse zu bewegen.

27. Vorrichtung nach Anspruch 1, wobei das Gehäuse so ausgeführt ist, dass es eine Vielzahl von Elektroden trägt, und wobei die Vorrichtung des Weiteren ein Betätigungselement umfasst, das relativ zu dem Gehäuse bewegt werden kann, um nacheinander mit jeder der Vielzahl von Elektroden einzeln von außerhalb des Gehäuses in Eingriff zu kommen und sie zu bewegen, und die scharfe Spitze jeder Elektrode darunter in dem Gewebe zu platzieren.

28. Vorrichtung nach Anspruch 28, die des Weiteren ein Magazin in dem Gehäuse umfasst, das die Vielzahl von Elektroden trägt.

29. Vorrichtung nach Anspruch 1, die des Weiteren ein Betätigungselement umfasst, das lösbar mit der Elektrode gekoppelt ist, wobei das Betätigungselement und die Elektrode so ausgeführt sind, dass sie sich beim Piatzieren der scharfen Spitze der Elektrode in dem Gewebe in der gleichen Richtung bewegen.

30. Vorrichtung nach Anspruch 1, wobei das Gehäuse ein Druckelement enthält, das an die Elektrode angrenzend positioniert ist, um Druck auf das Gewebe an einen Elektroden-Einführpunkt angrenzend auszuüben, über den die Elektrode in das Gewebe eintritt.

31. Vorrichtung nach Anspruch 30, wobei das Druckelement sich beim Einführen der Elektrode vollständig um den Einführpunkt herum erstreckt.

32. Vorrichtung nach Anspruch 1, wobei das Gehäuse eine distale Fläche enthält, die so positioniert ist, dass sie mit einer Haut des Patienten in Kontakt kommt, und wobei das Gehäuse ein Druckelement enthält, das sich über die distale Fläche hinaus erstreckt, um Druck auf die Haut auszuüben, wobei das Druckelement eine Öffnung hat, die die Elektrode aufnimmt, wenn die Elektrode in das Gewebe hinein tritt.

33. Vorrichtung nach Anspruch 32, wobei das Druckelement einen ersten Durchmesser hat und die distale Fläche einen zweiten Durchmesser hat, der größer ist als der erste Durchmesser.

34. Vorrichtung nach Anspruch 1, wobei das Gehäuse undurchsichtig ist.

35. Vorrichtung nach Anspruch 1, wobei das Gehäuse so positioniert ist, dass es wenigstens die scharfe Spitze der Elektrode einschließt, wenn die Elektrode in einem nicht eingesetzten Zustand ist.

36. Vorrichtung nach Anspruch 1, wobei die scharfe Spitze der Elektrode in einem eingesetzten Zustand außerhalb des Gehäuses positioniert ist und wobei die Elektrode des Weiteren in dem Gehäuse positioniert ist, wenn die Elektrode in dem eingesetzten Zustand ist.

37. Vorrichtung nach Anspruch 1, wobei die Elektrode relativ zu dem Gehäuse zwischen einem eingesetzten Zustand und einem nicht eingesetzten Zustand bewegt werden kann und wobei das Gehäuse des Weiteren so positioniert ist, dass es wenigstens die scharfe Spitze der Elektrode einschließt, nachdem sich die Elektrode aus dem eingesetzten Zustand in einen nicht eingesetzten Zustand bewegt hat.

38. Vorrichtung nach Anspruch 1, wobei das Gehäuse eine Grenzfläche mit der Haut des Patienten bildet, wenn das Gehäuse mit der Haut in Kontakt ist, und das Gehäuse sowie die Haut die scharfe Spitze der Elektrode vollständig umgeben, wenn sich die Elektrode in das Gewebe hinein bewegt.

39. Vorrichtung nach Anspruch 1, wobei das Gehäuse des Weiteren eine Arretierbaugruppe enthält, die so positioniert ist, dass sie die relative Bewegung der Elektrode und des Gehäuses zueinander verhindert, wenn die Arretierbaugruppe in Eingriff ist.

40. Vorrichtung nach Anspruch 39, wobei die Arretierbaugruppe eine federgespannte Klinke enthält, die funktionell mit der Elektrode gekoppelt ist und mit einer entsprechenden Öffnung in dem Gehäuse in Eingriff gebracht werden kann.

41. Vorrichtung nach Anspruch 39, wobei die Arretierbaugruppe ein Betätigungselement enthält, das mit der Elektrode gekoppelt ist und eine Klinke hat, die lösbar mit einer Öffnung in dem Gehäuse in Eingriff gebracht werden kann, und wobei die Vorrichtung des Weiteren ein Instrument enthält, das so eingerichtet ist, dass es die Arretierbaugruppe löst und die scharfe Spitze der Elektrode aus dem Gehäuse bewegt.

42. Vorrichtung nach Anspruch 41, wobei das Instrument so ausgeführt ist, dass es die scharfe Spitze der Elektrode wieder in das Gehäuse bewegt, nachdem es die scharfe Spitze der Elektrode aus dem Gehäuse bewegt hat, und die Arretierbaugruppe in Eingriff bringt, um weitere relative Bewegung der Elektrode und des Gehäuses zueinander zu verhindern.

43. Vorrichtung nach Anspruch 41, wobei das Betätigungselement-instrument einen elektrischen Kontakt hat, der so positioniert ist, dass er elektrische Verbindung zwischen der Elektrode und der Steuereinheit herstellt.

44. Vorrichtung nach Anspruch 1, wobei das Gehäuse so eingerichtet ist, dass es keinen Teil der Elektrode einschließt, wenn die Elektrode in einem eingesetzten Zustand ist, in dem die scharfe Spitze der Elektrode in das Gewebe eingeführt worden ist.

45. Vorrichtung nach Anspruch 1, wobei das Gehäuse so ausgeführt ist, dass es eine Vielzahl von Elektroden enthält, und wobei die Vorrichtung des Weiteren ein Betätigungselement umfasst, das relativ zu dem Gehäuse bewegt werden kann, um jede Elektrode jeweils einzeln aus dem Gehäuse heraus zu bewegen.

46. Vorrichtung nach Anspruch 1, wobei das Gehäuse ein erstes Gehäuse ist und wobei die Vorrichtung des Weiteren ein zweites Gehäuse umfasst, das wenigstens das scharfe Ende der Elektrode einschließt, wenn sich die Elektrode aus einem eingesetzten Zustand in einen nicht eingesetzten Zustand bewegt hat.

47. Vorrichtung nach Anspruch 1, die des Weiteren einen Mechanismus zum Halten der eingesetzten Elektrode umfasst, der so ausgeführt ist, dass er die Elektrode nach dem Einführen der scharfen Spitze der Elektrode in das Gewebe trägt.

48. Vorrichtung nach Anspruch 1, die des Weiteren die Steuereinheit umfasst, wobei die Steuereinheit mit der Elektrode gekoppelt ist und wobei die Steuereinheit so ausgeführt ist, dass sie eine stromgeregelte und stromausgeglichene Wellenform zu der Elektrode leitet.

49. Ausziehvorrichtung für perkutane Elektroden, die umfasst:
ein Gehäuse, das so ausgeführt ist, dass es in einer menschlichen Hand gehalten wird, wobei das Gehäuse eine Öffnung an einem distalen Ende hat; und
ein Betätigungselement, das so ausgeführt wird, dass es mit der menschlichen Hand in Eingriff kommt, wobei das Betätigungselement relativ zu dem Gehäuse zwischen einer ersten Position, in der das Betätigungselement mit einer Elektrode gekoppelt ist, wenn die Elektrode in einen Patienten eingeführt ist, und einer zweiten Position bewegt werden kann, in der das Betätigungselement mit der Elektrode gekoppelt ist und die Elektrode über die Öffnung vollständig in das Gehäuse herausgezogen ist.

50. Ausziehvorrichtung nach Anspruch 49, die des Weiteren eine Elektroden-Eingriffs-Einrichtung umfasst, die mit dem Betätigungselement verbunden und so positioniert ist, dass sie mit einem freiliegenden Abschnitt einer Elektrode in Funktion in Eingriff kommt, wobei die Elektroden-Eingriffs-Einrichtung mit dem Betätigungselement zwischen der ersten Position und der zweiten Position bewegt werden kann.

51. Ausziehvorrichtung nach Anspruch 49, wobei das Betätigungselement so ausgeführt ist, dass es bei Bewegung zwischen der ersten und der zweiten Position mit einem menschlichen Daumen in Eingriff kommt.

52. Ausziehvorrichtung nach Anspruch 49, wobei das Gehäuse einen Halter für gebrauchte Elektroden enthält, der so positioniert ist, dass er eine Vielzahl von Elektroden trägt, die durch Funktion des Betätigungselementes in das Gehäuse hinein bewegt worden sind.

53. Ausziehvorrichtung nach Anspruch 49, die des Weiteren umfasst:
einen Arm, der schwenkbar mit dem Betätigungselement gekoppelt ist und relativ zu einer Achse, die sich durch die Öffnung hindurch erstreckt, zwischen einer fluchtenden Position, einer ersten nicht fluchtenden Position auf einer Seite der Achse und einer zweiten nicht fluchtenden Position auf einer anderen Seite der Achse bewegt werden kann;
eine Arm-Feder, die mit dem Arm und dem Gehäuse gekoppelt ist, um den Arm in die fluchtende Position zu spannen;
ein Nockenelement nahe an dem Arm, wobei das Nockenelement eine erste Nockenfläche hat, die mit dem Arm in Eingriff ist, wenn sich das Betätigungselement aus der ersten Position in die zweite Position bewegt, um den Arm aus der fluchtenden Position in die erste nicht fluchtende Position zu bewegen, und das Nockenelement des Weiteren eine zweite Nockenfläche hat, die mit dem Arm in Eingriff ist, wenn sich das Betätigungselement aus der ersten Position in die zweite Position bewegt;
eine Betätigungselement-Feder, die mit dem Betätigungselement und dem Gehäuse gekoppelt ist, um das Betätigungselement in die zweite Position zu spannen; und
eine Eingriffsgabel, die mit dem Arm verbunden ist und zwei Zinken hat, die so positioniert sind, dass sie um die Elektrode herum passen und mit der Elektrode in Eingriff sind, wenn sich das Betätigungselement in der ersten Position befindet.

## Revendications

1. Dispositif servant à administrer une thérapie électrique percutanée, comprenant :
une électrode (1,12,34) raccordable électriquement à une unité de commande pour fournir une thérapie électrique à un patient au cours d'une opération, l'électrode ayant une première extrémité et une seconde extrémité opposée à la première extrémité, la première extrémité ayant une extrémité (2,20,38) pointue configurée pour être insérée dans le tissu du patient ;
un déclencheur couplé à l'électrode pour porter l'électrode ; et
un logement d'électrode (4,18,40,36) couplé de manière opérationnelle au déclencheur avec le déclencheur étant reçu dans le logement d'électrode, le logement d'électrode étant placé pour soutenir le déclencheur pendant l'insertion de l'électrode dans le tissu, le logement portant le déclencheur pour guider le déclencheur et l'électrode le long d'un chemin et/ou pour commander l'accès à l'électrode, le déclencheur étant mobile par rapport au logement d'électrode entre une première position avec l'électrode placée au moins partiellement à l'intérieur du logement et une seconde position avec l'électrode placée au moins partiellement à l'extérieur du logement.

2. Dispositif selon la revendication 1, comprenant en outre l'unité de commande, l'unité de commande étant couplée à l'électrode, et dans lequel l'unité de commande est configurée pour diriger vers l'électrode une forme d'onde avec courant régulé et courant équilibré avec une amplitude allant jusqu'à approximativement 20 mA et une fréquence à partir d'environ 4 Hz jusqu'à environ 50 Hz.

3. Dispositif selon la revendication 1, dans lequel le logement a une face distale configurée pour être placée sur le patient pour soutenir l'électrode par rapport au patient et pour commander un angle entre le logement et le patient, le logement ayant un premier diamètre sur la face distale et un second diamètre espacé de la face distale, le premier diamètre étant plus grand que le second diamètre.

4. Dispositif selon la revendication 1, dans lequel le logement a une face distale configurée pour être placée sur le patient pour soutenir l'électrode par rapport au patient, et dans lequel la face distale a une ouverture (5,24,41) configurée pour entourer au moins une partie de l'électrode pendant l'insertion de l'électrode dans le tissu.

5. Dispositif selon la revendication 1, comprenant en outre un raccordement mécanique entre l'électrode et le logement pendant l'insertion de l'électrode dans le tissu, le raccordement mécanique comprenant une cheville (70) couplée de manière opérationnelle à l'électrode et placée de manière coulissante dans une cannelure (74) du logement, la cannelure étant placée parallèlement au chemin le long duquel l'électrode est guidée, le chemin comprenant un axe linéaire à un angle fixe par rapport au patient quand le logement est engagé dans le patient.

6. Dispositif selon la revendication 1, dans lequel l'électrode et le logement sont configurés pour rester reliés de manière mécanique au cours de l'application de la thérapie électrique au patient.

7. Dispositif selon la revendication 1, dans lequel le logement est configuré pour être séparé de l'électrode après l'insertion de l'électrode dans le tissu mais avant l'application,d'une thérapie électrique au patient.

8. Dispositif selon la revendication 1, comprenant en outre un élément d'alignement du logement (140) disposé sur le tissu et adapté pour interagir mécaniquement avec le logement afin d'aligner le logement par rapport au tissu.

9. Dispositif selon la revendication 1, comprenant en outre un élément d'alignement du logement disposé sur le tissu et adapté pour interagir mécaniquement avec le logement afin d'aligner le logement par rapport au tissu, l'élément d'alignement du logement comprenant une pièce reliée au tissu.

10. Dispositif selon la revendication 9, dans lequel la pièce comprend une partie compressible ayant un adhésif à attacher au patient, la pièce ayant en outre un élément annulaire globalement rigide placé pour engager de manière détachable le logement et pour entourer au moins partiellement l'électrode pendant l'insertion de l'électrode dans le tissu.

11. Dispositif selon la revendication 10, dans lequel l'électrode comprend des première et seconde brides espacées se prolongeant vers l'extérieur vers la seconde extrémité, et dans lequel l'élément annulaire rigide comprend une lèvre se prolongeant vers l'intérieur placée pour s'adapter entre les première et seconde brides quand l'électrode est insérée dans le tissu à une distance choisie.

12. Dispositif selon la revendication 1, comprenant en outre un déclencheur (12) d'électrode mobile dans le logement, le déclencheur d'électrode étant placé pour faire entrer l'extrémité pointue de l'électrode dans le tissu du patient.

13. Dispositif selon la revendication 1, dans lequel le logement comprend un canal (42) disposé de manière annulaire autour de l'électrode et placé pour être au contact et guider au moins une partie de l'électrode au cours de l'opération pendant que l'électrode se déplace par rapport au logement dans le tissu.

14. Dispositif selon la revendication 1, comprenant en outre une butée d'arrêt dépendant du logement et placée pour se coupler mécaniquement de manière détachable à l'électrode et limiter une longueur de déplacement de l'électrode pendant que l'électrode se déplace par rapport au logement.

15. Dispositif selon la revendication 1, comprenant en outre :
un déclencheur d'électrode relié à l'électrode et mobile avec l'électrode par rapport au logement ; et
une butée d'arrêt dépendant du logement et placée pour être au contact du déclencheur d'électrode et pour arrêter le mouvement du déclencheur d'électrode quand l'électrode atteint une profondeur choisie dans le tissu.

16. Dispositif selon la revendication 1, comprenant en outre :
un déclencheur d'électrode relié à l'électrode et mobile avec l'électrode par rapport au logement ; et
un instrument déclencheur relié de manière amovible au déclencheur pour déplacer le déclencheur et l'électrode par rapport au tissu.

17. Dispositif selon la revendication 16, dans lequel l'instrument déclencheur a un contact électrique placé pour venir au contact de l'électrode, de manière amovible, et pour établir une communication électrique entre l'électrode et l'unité de commande.

18. Dispositif selon la revendication 1, comprenant en outre :
un élément de glissière placé de manière coulissante dans le logement et coulissant par rapport au logement pour venir au contact de l'électrode, insérer l'électrode dans le patient et libérer l'électrode ; et
une butée d'arrêt dépendant du logement et placée pour être au contact de l'élément de glissière quand l'électrode atteint une profondeur choisie dans le tissu.

19. Dispositif selon la revendication 18, comprenant en outre un déclencheur couplé de manière mobile au logement et couplé à l'élément de glissière avec un ensemble de transmission placé pour insérer l'électrode à une profondeur prédéterminée dans le tissu quand le déclencheur se déplace à une distance prédéterminé du déclencheur, la profondeur prédéterminée étant plus grande que la distance prédéterminée du déclencheur.

20. Dispositif selon la revendication 19, dans lequel l'ensemble de transmission comprend un premier support couplé de manière opérationnelle au déclencheur, un second support couplé de manière opérationnelle à l'élément de glissière et un ensemble d'engrenage placé entre les premier et second supports et rotatif autour d'un axe de rotation, l'ensemble d'engrenage ayant les premières dents engagées dans le premier support et placées à un premier diamètre depuis l'axe de rotation, l'ensemble d'engrenage ayant des deuxièmes dents concentriques avec les premières dents, engagées dans le second support et placées à un second diamètre depuis l'axe de rotation, le second diamètre étant plus grand que le premier diamètre pour transmettre un premier mouvement linéaire du déclencheur en un second mouvement linéaire de l'électrode, le second mouvement étant plus grand que le premier mouvement.

21. Dispositif selon la revendication 1, comprenant en outre un mécanisme porteur d'une électrode déployée, configuré pour maintenir l'électrode en place après l'insertion de l'extrémité pointue de l'électrode dans le tissu du patient.

22. Dispositif selon la revendication 1, dans lequel l'électrode comprend une poignée d'électrode placée pour se prolonger à l'extérieur du tissu après l'insertion de l'extrémité pointue dans le tissu.

23. Système selon la revendication 1, dans lequel le logement est configuré, pour être relié de manière amovible au patient.

24. Système selon la revendication 1, comprenant en outre un conducteur configuré pour se relier entre l'électrode et l'unité de commande, dans lequel le logement est configuré pour soutenir le conducteur et pour fournir un réducteur de tension à l'électrode.

25. Dispositif selon la revendication 1, dans lequel l'électrode comprend une partie de connecteur électrique vers la seconde extrémité, la partie de connecteur de l'électrode étant exposée au-dessus de la peau du patient quand l'extrémité pointue de l'électrode est dans le tissu.

26. Dispositif selon la revendication 1, comprenant en outre un déclencheur configuré pour être en contact avec un pouce humain pour déplacer le déclencheur et l'électrode par rapport au logement.

27. Dispositif selon la revendication 1, dans lequel le logement est configuré pour soutenir une pluralité d'électrodes, et dans lequel l'appareil comprend en outre un déclencheur mobile par rapport au logement pour successivement pour venir au contact et déplacer chacune de la pluralité d'électrodes individuellement vers l'extérieur du logement afin de placer l'extrémité pointue de chaque électrode sous le tissu.

28. Dispositif selon la revendication 27, comprenant en outre un magasin dans le logement et soutenant la pluralité d'électrodes.

29. Dispositif selon la revendication 1, comprenant en outre un déclencheur couplé de manière détachable à l'électrode, le déclencheur et l'électrode étant configurés pour se déplacer dans la même direction pendant le placement de l'extrémité pointue de l'électrode dans le tissu.

30. Dispositif selon la revendication 1, dans lequel le logement comprend un élément de pression placé à côté de l'électrode pour fournir une pression contre le tissu à côté d'un point d'insertion d'électrode à travers lequel l'électrode entre dans le tissu.

31. Dispositif selon la revendication 30, dans lequel l'élément de pression se prolonge complètement autour du point d'insertion pendant l'insertion de l'électrode.

32. Dispositif selon la revendication 1, dans lequel le logement comprend une face distale placée pour contacter une peau du patient, et dans lequel le logement comprend un élément de pression se prolongeant au-delà de la face distale pour fournir une pression sur la peau, l'élément de pression ayant une ouverture pour recevoir l'électrode lorsque l'électrode entre dans le tissu.

33. Dispositif selon la revendication 32, dans lequel l'élément de pression a un premier diamètre et la face distale a un second diamètre plus grand que le premier diamètre.

34. Dispositif selon la revendication 1, dans lequel le logement est opaque.

35. Dispositif selon la revendication 1, dans lequel le logement est placé pour contenir au moins l'extrémité pointue de l'électrode quand l'électrode est dans un état non déployé.

36. Dispositif selon la revendication 1, dans lequel l'extrémité pointue de l'électrode est placée à l'extérieur du logement dans un état déployé et en outre dans lequel une partie de l'électrode est placée à l'intérieur du logement quand l'électrode est dans un état déployé.

37. Dispositif selon la revendication 1, dans lequel l'électrode est mobile par rapport au logement entre un état déployé et un état non déployé, et en outre dans lequel le logement est placé pour contenir au moins l'extrémité pointue de l'électrode après que l'électrode est passée de l'état déployé à un état non déployé.

38. Dispositif selon la revendication 1, dans lequel le logement forme une interface avec la peau du patient quand le logement est en contact avec la peau, le logement et la peau entourant complètement l'extrémité pointue de l'électrode pendant que l'électrode entre dans le tissu.

39. Dispositif selon la revendication 1, dans lequel le logement comprend en outre un ensemble de verrouillage placé pour empêcher le mouvement relatif entre l'électrode et le logement quand l'ensemble de verrouillage est enclenché.

40. Dispositif selon la revendication 39, dans lequel l'ensemble de verrouillage comprend une détente sous tension d'un ressort couplée de manière opérationnelle à l'électrode et pouvant être enclenchée avec une ouverture correspondante dans le logement.

41. Dispositif selon la revendication 39, dans lequel l'ensemble de verrouillage comprend un déclencheur couplé à l'électrode et ayant une détente pouvant être enclenchée de manière détachable avec une ouverture dans le logement, et dans lequel le dispositif comprend en outre un instrument adapté pour libérer l'ensemble de verrouillage et pour déplacer l'extrémité pointue de l'électrode hors du logement.

42. Dispositif selon la revendication 41, dans lequel l'instrument est configuré pour déplacer l'extrémité pointue de l'électrode de nouveau dans le logement après avoir déplacé l'extrémité pointue de l'électrode hors du logement et pour enclencher l'ensemble de verrouillage afin d'empêcher davantage de mouvement relatif entre l'électrode et le logement.

43. Dispositif selon la revendication 41, dans lequel l'instrument déclencheur a un contact électrique placé pour établir une communication électrique entre l'électrode et une unité de commande.

44. Dispositif selon la revendication 1, dans lequel le logement est adapté pour ne contenir aucune des électrodes quand l'électrode est dans un état déployé dans lequel l'extrémité pointue de l'électrode a été insérée dans le tissu.

45. Dispositif selon la revendication 1, dans lequel le logement est configuré pour contenir une pluralité d'électrodes et dans lequel le dispositif comprend en outre un déclencheur mobile par rapport au logement pour déplacer chaque électrode hors du logement une par une.

46. Dispositif selon la revendication 1, dans lequel le logement est un premier logement et dans lequel le dispositif comprend en outre un second logement pour contenir au moins l'extrémité pointue de l'électrode quand l'électrode est passée d'un état déployé à un état non déployé.

47. Dispositif selon la revendication 1, comprenant en outre un mécanisme portant une électrode déployée configuré pour maintenir l'électrode en place après l'insertion de l'extrémité pointue de l'électrode dans le tissu.

48. Dispositif selon la revendication 1, comprenant en outre l'unité de commande, l'unité de commande étant couplée à l'électrode, et dans lequel l'unité de commande est configurée pour diriger vers l'électrode une forme d'onde avec courant régulé et courant équilibré.

49. Extracteur d'électrode percutanée comprenant :
un logement configuré pour être tenu dans une main humaine, le logement ayant une ouverture à une extrémité distale ; et
un déclencheur configuré pour être enclenché par la main humaine, le déclencheur étant mobile par rapport au logement entre une première position avec le déclencheur couplé à une électrode tandis que l'électrode est insérée dans un patient, et une seconde position avec le déclencheur couplé à l'électrode et l'électrode retirée par l'ouverture et complètement à l'intérieur du logement.

50. L'extracteur selon la revendication 49, comprenant en outre un enclencheur d'électrode relié au déclencheur et placé pour être au contact une partie exposée d'une électrode au cours de l'opération, l'enclencheur d'électrode étant mobile avec le déclencheur entre la première position et la seconde position.

51. L'extracteur selon la revendication 49, dans lequel le déclencheur est configuré pour être au contact d'un pouce humain pendant le mouvement entre les première et seconde positions.

52. L'extracteur selon la revendication 49, dans lequel le logement comprend un support d'électrode utilisé, placé pour soutenir une pluralité d'électrodes qui ont été déplacées dans le logement par l'actionnement du déclencheur.

53. L'extracteur selon la revendication 49, comprenant en outre :
un bras couplé de manière pivotante au déclencheur et mobile par rapport à un axe s'étendant à travers l'ouverture entre une position alignée, une première position non alignée d'un côté de l'axe, et une seconde position non alignée d'un autre côté de l'axe ;
un ressort de bras couplé entre le bras et le logement pour polariser le bras dans la position alignée ;
un élément de came proche du bras, l'élément de came ayant une première surface de came au contact du bras quand le déclencheur se déplace de la seconde position à la première position pour déplacer le bras de la position alignée à la première position non alignée, l'élément de came ayant en outre une seconde surface de came au contact du bras quand le déclencheur se déplace de la première position à la seconde position ;
un ressort de déclencheur couplé entre le déclencheur et le logement pour rappeler le déclencheur dans la deuxième position ; et
une fourche d'enclenchement reliée au bras et ayant deux dents placées pour s'adapter autour de l'électrode et pour enclencher l'électrode quand le déclencheur est en première position.
